# EUROPEAN PATENT APPLICATION

(11) **EP 3 539 562 A1**
(43) Date of publication of application: **18.09.2019**
(21) Application number: 18161259.9
(22) Date of filing: 12.03.2018
(51) Int. Cl.: A61K 39/00, A61K 39/12

(54) **IMMUNOTHERAPEUTIC PEPTIDES**

(71) Applicant: Eberhard Karls Universität Tübingen Medizinische Fakultät, 72074 Tübingen (DE)
(72) Inventor: TABATABAI, Ghazaleh, 72076 Tübingen (DE); RAJARAMAN, Srinath, 72072 Tübingen (DE); GHOSH, Michael, 71083 Herrenberg (DE)
(74) Representative: Witte, Weller & Partner Patentanwälte mbB

(57) **Abstract**

The present invention relates to a pharmaceutical composition for the treatment and/or prophylaxis of a disease, preferably a tumor disease, and peptides being comprised by the pharmaceutical composition, a nucleic acid encoding said peptides, an expression vector capable of expressing said nucleic acid, a recombinant host cell comprising said peptides or expression vector, an antibody and T cell receptor and chimeric antigen receptor T cells which specifically recognize said peptides, and to a method for the treatment and/or the prophylaxis of a disease, preferably a tumor disease.

## Description

The present invention relates to a pharmaceutical composition for the treatment and/or prophylaxis of a disease, preferably a tumor disease, and peptides being comprised by the pharmaceutical composition, a nucleic acid encoding said peptides, an expression vector capable of expressing said nucleic acid, a recombinant host cell comprising said peptides or expression vector, an antibody and T cell receptor and chimeric antigen receptor T cells which specifically recognize said peptides, and to methods for the treatment and/or the prophylaxis of a disease, preferably a tumor disease.

### FIELD OF THE INVENTION

The invention relates to the field of molecular biology and molecular medicine, in particular the treatment of diseases, such as cancer.

### BACKGROUND OF THE INVENTION

In 2008, approximately 12.7 million cancers were diagnosed and in 2010 nearly 7.98 million people died. Cancers account for approximately 13% of deaths. The most common are lung cancer (1.4 million deaths), stomach cancer (740,000), liver cancer (700,000), colorectal cancer (610,000) and breast cancer (460,000). This makes invasive cancer the leading cause of death in the developed world and the second leading in the developing world.

Many treatment options for cancer exist. The primary ones include surgery, chemotherapy, radiation therapy, hormonal therapy, targeted therapy and palliative care. Which treatments are used depends on the type, location and grade of the cancer as well as the patient's health and preferences. The treatment intent may or may not be curative.

Cancer immunotherapy is the use of the immune system to treat cancer. This approach exploits the fact that cancer cells often have molecules on their surface that can be detected by the immune system, known as tumor-associated antigens (TAAs); they are often proteins, peptides or other macromolecules. One form of cancer immunotherapy involves the use of cancer vaccine. An approach to cancer vaccination is to separate proteins from cancer cells and immunize patients against those proteins as antigens, in the hope of stimulating the immune system to kill the cancer cells. Research on cancer vaccines is underway for treatment of breast, lung, brain, colon, skin, kidney, prostate and other cancers. Another approach is to generate an immune response in situ in the patient using oncolytic viruses, i.e. viruses that preferentially infects and kills cancer cells. A number of viruses including adenovirus, reovirus, measles, Newcastle disease virus and vaccinia have now been clinically tested as oncolytic agents. Herpes simplex virus is even an FDA approved composition marketed as Imlygic (talimogene la-herparepvec) for treatment of melanoma.

Therefore, TAAs are a starting point for the development of tumor vaccines. The methods for identifying and characterizing the TAAs are usually based on the use of T cells that can be isolated from patients or healthy subjects, or they are based on the generation of differential transcription profiles or differential peptide expression patterns between tumors and normal tissues. However, the identification of genes overexpressed in tumor tissues or human tumor cell lines, or selectively expressed in such tissues or cell lines, does not provide precise information as to the use of the antigens being transcribed from these genes in an immune therapy. This is because only an individual subpopulation of epitopes of these antigens are suitable for such an application since a T cell with a corresponding TCR has to be present and the immunological tolerance for this particular epitope needs to be absent or minimal.

In spite of significant progress in basic and clinical research concerning TAAs only limited number of candidate TAAs for the treatment of cancer are available. TAAs abundantly expressed in cancer cells, and at the same time which expression is restricted to cancer cells would be promising candidates as immunotherapeutic targets. Further, identification of new TAAs inducing potent and specific antitumor immune responses is expected to encourage clinical use of peptide vaccination strategy in various types of cancer.

Furthermore, although advances have been made in the development of molecular-targeting drugs for cancer therapy, the ranges of tumor types that respond as well as the effectiveness of the treatments are still very limited. Hence, it is urgent to develop new anti-cancer agents that target molecules highly specific to malignant cells and are likely to cause minimal or no adverse reactions.

Prior art document WO 2017/157972 discloses peptides and combinations of peptides for use in immunotherapy against non-small cell lung cancer and other cancers. US 6,013,263 discloses measles virus peptides with antifusiogenic and antiviral activities. WO 2008/086042 suggests the use of a measles or respiratory syncytial viruses to prevent infections. US 2010/0322968 proposes a peptide vaccine for the treatment of cancer and viral infections.

Against this background it is an object underlying the invention to provide a new therapeutic means for the treatment and/or prophylaxis of a disease, preferably a tumor disease, to increase the available therapeutic tools and approaches for effectively combating diseases like cancer, especially glioblastoma.

### SUMMARY OF THE INVENTION

This object is met by the provision of a pharmaceutical composition for the treatment and/or prophylaxis of a disease comprising a first peptide derived from the RNA-directed RNA polymerase L of the Measles virus (MeV-L polymerase).

The inventors have surprisingly found that Measles virus derived peptides are presented in the HLA ligandome of tumor cells after an infection with oncolytic measles viruses has occured. This results in the tumor cells being recognizable to the immune system and thus distinguishable from normal tissue. Noteworthy and completely new is the finding that the presented peptides represent a region of the so-called RNA-directed RNA polymerase L of the measles vaccine virus, an area that is essential for the measles virus and therefore highly conserved. The high conservation of this region confers the power on the new pharmaceutical composition not only to treat a particular disease, such as a specific type of cancer, but a large number of various and different diseases and tumor entities.

A "pharmaceutical composition" according to the invention is a composition suitable for administration to a human being in a medical setting. Preferably, a pharmaceutical composition is sterile and produced according to GMP guidelines.

The RNA-directed RNA polymerase of the Measles virus (MeV) is a Measles virus encoded enzyme that catalyzes the transcription of viral mRNAs, their capping and polyadenylation and the replication of viral genomic RNA. It is comprised of two viral polypeptides, the large (L) and the phospho- (P) proteins. The L protein (MeV-L polymerase) (UniProtKB - P12576 (L_MEASE), also called protein L, large structural protein, replicase of transcriptase, includes the following 4 domains: RNA-directed RNA polymerase (Enzyme Commission number (EC): 2.7.7.48), mRNA (guanine-N(7)-)-methyltransferase (EC: 2.1.1.56), GDP polyribonucleotidyltransferase (EC: 2.7.7.88), and Cap-specific mRNA (nucleoside-2'-O-)-methyltransferase 2 (EC: 2.1.1.296). It is encoded by the L gene of the virus.

The term "peptide" is used herein to designate a series of amino acid residues, connected one to the other typically by peptide bonds between the alpha-amino and carbonyl groups of the adjacent amino acids. The peptides are preferably 9 amino acids in length, but can be as short as 5, 6, 7, or 8 amino acids in length, and as long as 10, 11, 12, 13, 14, 15, 16, 17, 18, 19 or 20 or more amino acids in length.

Furthermore, the term "peptide" shall include salts of a series of amino acid residues, connected one to the other typically by peptide bonds between the alpha-amino and carbonyl groups of the adjacent amino acids. Preferably, the salts are pharmaceutical acceptable salts of the peptides, such as, for example, the chloride or acetate (trifluoroacetate) salts. It has to be noted that the salts of the peptides according to the present description may differ substantially from the peptides in their state(s) in vivo, as the peptides are not salts in vivo.

According to the invention, a peptide is "derived" from the MeV-L polymerase if its amino acid sequence shares a degree of identity with an amino acid sequence of the MeV-L polymerase that is ≥ 85%, preferably ≥ 90%, further preferably ≥ 95%, ≥ 96%, ≥ 97%, ≥ 98%, ≥ 99%, highly preferably = 100%. In the latter case the peptide according to the invention represents a portion, segment or fragment of the MeV-L polymerase.

In accordance with the present invention, the term "percent identity" or "percent identical", when referring to a sequence, means that a sequence is compared to a claimed or described sequence after alignment of the sequence to be compared (the "Compared Sequence", e.g. the amino acid sequence of the peptides according to the invention) with the described sequence (the "Reference Sequence", e.g. the amino acid sequence of the MeV-L polymerase). The percent identity is then determined according to the following formula: percent identity = 100 [1 -(C/R)], wherein C is the number of differences between the Reference Sequence and the Compared Sequence over the length of alignment between the Reference Sequence and the Compared Sequence, wherein (i) each amino acid in the Reference Sequence that does not have a corresponding aligned amino acid in the Compared Sequence and, (ii) each gap in the Reference Sequence and (iii) each aligned amino acid in the Reference Sequence that is different from an aligned amino acid in the Compared Sequence, constitutes a difference and (iv) the alignment has to start at position 1 of the aligned sequences; and R is the number of amino acids in the Reference Sequence over the length of the alignment with the Compared Sequence with any gap created in the Reference Sequence also being counted as an amino acid. If an alignment exists between the Compared Sequence and the Reference Sequence for which the percent identity as calculated above is about equal to or greater than a specified minimum Percent Identity then the Compared Sequence has the specified minimum percent identity to the Reference Sequence even though alignments may exist in which the herein above calculated percent identity is less than the specified percent identity.

As used herein, the terms "portion", "segment" and "fragment", when used in relation to the peptides according to the invention, refer to a continuous sequence of amino acid residues, which sequence forms a subset of a larger of amino acid residues sequence, e.g. of the amino acid residues of the MeV-L polymerase.

According to the invention "disease" includes any kind of pathological state of an organism, especial humans, including and preferably tumor diseases such as glioblastoma, and infectious diseases such as measles.

According to the invention "treatment" refers to the therapy of a disease whereas "prophylaxis" refers to the prevention of a disease.

The pharmaceutical composition according to the invention may also include one or more adjuvants. Adjuvants are substances that non-specifically enhance or potentiate the immune response, e.g., immune responses mediated by CD8-positive T-cells and helper-T (TH) cells to an antigen, and would thus be considered useful in the medicament of the present description. Suitable adjuvants include, but are not limited to, 1018 ISS, aluminum salts, AMPLIVAX®, AS15, BCG, CP-870,893, CpG7909, CyaA, dSLIM, fla- gellin or TLR5 ligands derived from flagellin, FLT3 ligand, GM-CSF, IC30, IC31 , Imiquimod (ALDARA®), resiquimod, ImuFact IMP321, Interleukins as IL-2, IL-13, IL- 21, Interferon-alpha or -beta, or pegylated derivatives thereof, IS Patch, ISS, ISCOMATRIX, ISCOMs, Juvlmmune®, LipoVac, MALP2, MF59, monophosphoryl lipid A, Montanide IMS 1312, Montanide ISA 206, Montanide ISA 50V, Montanide ISA-51, water-in-oil and oil-in-water emulsions, OK-432, OM-174, OM-197-MP-EC, ONTAK, OspA, PepTel® vector system, poly(lactid co-glycolid) [PLG]-based and dextran microparticles, talactoferrin SRL172, Virosomes and other Virus-like particles, YF-17D, VEGF trap, R848, beta-glucan, Pam3Cys, Aquila's QS21 stimulon, which is derived from saponin, mycobac-terial extracts and synthetic bacterial cell wall mimics, and other proprietary adjuvants such as Ribi's Detox, Quil, or Superfos. Adjuvants such as Freund's or GM-CSF are preferred. Several immunological adjuvants (e.g., MF59) specific for dendritic cells and their preparation have been described previously (Allison and Krummel, 1995). Also cytokines may be used. Several cytokines have been directly linked to influencing dendritic cell migration to lymphoid tissues, e.g., TNF, accelerating the maturation of dendritic cells into efficient antigen- presenting cells for T-lymphocytes, e.g., GM-CSF, IL-1 and IL-4, and acting as immunoadjuvants, e.g., IL-12, IL-15, IL-23, IL-7, IFN-alpha. IFN-beta. Other examples for useful adjuvants include, but are not limited to chemically modified CpGs, e.g., CpR, Idera, dsRNA analogues such as Poly(I:C) and derivates thereof, e.g., AmpliGen®, Hiltonol®, poly-(ICLC), poly(IC-R), poly(I:C12U), non-CpG bacterial DNA or RNA as well as immunoactive small molecules and antibodies such as cyclophosphamide, sunitinib, Bevacizumab®, Celebrex, NCX-4016, sildenafil, tadalafil, vardenafil, sorafenib, temozolomide, temsirolimus, XL-999, CP-547632, pazopanib, VEGF Trap, ZD2171, AZD2171, anti-CTLA4, other antibodies targeting key structures of the immune system (e.g., anti-CD40, anti-TGFbeta, anti-TNFalpha receptor) and SC58175, which may act therapeutically and/or as an adjuvant. The amounts and concentrations of adjuvants and additives useful in the context of the present description can readily be determined by the skilled artisan without undue experimentation.

The pharmaceutical composition may comprise a pharmaceutically acceptable, preferably aqueous carrier. In addition, the composition can contain excipients, such as buffers, binding agents, blasting agents, diluents, flavors, lubricants, etc. An extensive listing of excipients that can be used in such a composition, can be, for example, taken from A. Kibbe, Handbook of Pharmaceutical Excipients (4th edition, 2003).

The object underlying the invention is herewith completely solved.

According to an embodiment said first peptide is derived from the MeV-L polymerase of the Edmondston strain of MeV.

This measure has the advantage that such a first peptide is used which was exemplarily identified by the inventors. In the course of their experiments which resulted in the provision of the pharmaceutical composition and the peptides of the invention the inventors made use of the Edmondston strain of MeV.

In another embodiment of the invention said first peptide comprises at least nine sequentially concatenated amino acids derived from the MeV-L polymerase.

The inventors realized that a consecutive amino acid sequence of at least nine amino acids provides an epitope of the MeV-L polymerase which is sufficiently large to initiate an immune response directed to the disease causing agent such as the tumor cell.

According to another embodiment of the invention said first peptide comprises the following amino acid sequence: X1-Y-P-R-Y-S-N-F-X2, wherein X1 and X2 are selected from the group consisting of: V (Val), I (IIe), and L (Leu), wherein peptide VYPRYSNFI or Val-Tyr-Pro-Arg-Tyr-Ser-Asn-Phe-Ile (SEQ ID NO: 1) is of particular preference.

This measure has the advantage that such a peptide is used which is particularly suitable for treatment and/or prophylaxis of a disease. The afore-mentioned first peptides include the following amino acid sequences:
VYPRYSNFI (SEQ ID NO: 1);
VYPRYSNFL (SEQ ID NO: 2);
IYPRYSNFI (SEQ ID NO: 3);
LYPRYSNFI (SEQ ID NO: 4);
LYPRYSNFL (SEQ ID NO: 5);
IYPRYSNFL (SEQ ID NO: 6);
VYPRYSNFV (SEQ ID NO: 7);
IYPRYSNFV (SEQ ID NO: 8);
IYPRYSNFV (SEQ ID NO: 9);
LYPRYSNFV (SEQ ID NO: 10).

In another embodiment of the invention the pharmaceutical composition comprises a second peptide comprising the following amino acid sequence: *X₁*Y-T-*X₂-*R-T-*X₃*-K-*X₄*, wherein *X₁* is selected from the group consisting of: L, I, and V; *X₂* is D or E; *X₃* is E or D; and *X₄* is selected from the group consisting of: L, I, and V, wherein peptide LYTDRTEKL or Leu-Tyr-Thr-Asp-Arg-Thr-Glu-Lys-Leu (SEQ ID NO: 11) is of particular preference.

The inventors were able to realize that by the inclusion of said second peptide the therapeutic/prophylactic power of the pharmaceutical composition is increased. Said second peptide was processed from transforming growth factor-beta-induced (TGFBI).

The afore-mentioned second peptides include the following amino acid sequences:
LYTDRTEKL (SEQ ID NO: 11);
IYTDRTEKL (SEQ ID NO: 12);
LYTDRTEKI (SEQ ID NO: 13);
IYTDRTEKI (SEQ ID NO: 14);
LYTERTEKL (SEQ ID NO: 15);
LYTDRTDKL (SEQ ID NO: 16);
LYTERTDKL (SEQ ID NO: 17);
IYTERTEKL (SEQ ID NO: 18);
IYTDRTDKL (SEQ ID NO: 19);
IYTERTDKL (SEQ ID NO: 20);
LYTERTEKI (SEQ ID NO: 21);
LYTDRTDKI (SEQ ID NO: 22);
LYTERTDKI (SEQ ID NO: 23);
IYTERTEKI (SEQ ID NO: 24);
IYTDRTDKI (SEQ ID NO: 25);
IYTERTDKI (SEQ ID NO: 26);
VYTDRTEKL (SEQ ID NO: 27);
IYTDRTEKV (SEQ ID NO: 28);
LYTDRTEKV (SEQ ID NO: 29);
VYTDRTEKV (SEQ ID NO: 30);
IYTERTEKV (SEQ ID NO: 31);
LYTERTEKV (SEQ ID NO: 32);
VYTERTEKV (SEQ ID NO: 33);
VYTERTEKI (SEQ ID NO: 34);
VYTDRTDKV (SEQ ID NO: 35);
IYTDRTDKV (SEQ ID NO: 36);
LYTDRTDKV (SEQ ID NO: 37);
VYTDRTDKL (SEQ ID NO: 38);
VYTDRTDKI (SEQ ID NO: 39);
VYTERTDKV (SEQ ID NO: 40);
IYTERTDKV (SEQ ID NO: 41);
LYTERTDKV (SEQ ID NO: 42);
VYTERTDKI (SEQ ID NO: 43);
VYTERTDKL (SEQ ID NO: 44);

The inventors identified the amino acid sequences SEQ ID NO: 1 and SEQ ID NO: 11 by sequencing first and second peptides which were isolated from in vitro experiments whereas the further amino acid sequences were developed in silico by exchanging at the indicated positions the amino acid in the isolated peptides against amino acids with similar physico-chemical properties.

In another embodiment of the invention said disease is a tumor disease, preferably a glioblastoma.

This measure has the advantage that the pharmaceutical composition according to the invention is configured for a therapeutic use against such a kind of disease where an optimum of therapeutic activity can be achieved. The inventors identified the first and second peptides being presented in the HLA ligandome of glioblastoma cells which might by the reason for this therapeutic preference.

In another embodiment the pharmaceutical composition according to the invention is a tumor vaccine, preferably a vaccine against glioblastoma.

Said measure has the advantage that such a pharmaceutical composition is provided which allows an effective immunization of a patient in need against tumor cells, especially glioblastoma cells.

In another embodiment of the invention the pharmaceutical composition is configured for an administration to a patient in need, preferably to a patient with an HLA type HLA-A*24, further preferably with an HLA type HLA-A*24:03, further preferably it is configured for an administration after said patient has been treated with an oncolytic Measles vaccination virus, and further preferably it is configured for an administration followed by the additional administration of an immune checkpoint inhibitor.

The inventors have realized that a particular high effectivity of the pharmaceutical composition can be achieved in a patient with an HLA type HLA-A*24, further preferably with an HLA type HLA-A*24:03. The best effect is observed if, before the administration of the pharmaceutical composition, said patient has been treated with an oncolytic Measles vaccination virus. A further increase of the therapeutic effect is achieved if, after the administration of the pharmaceutical composition, an immune checkpoint inhibitor is administered to the patient. Non-limiting examples of suitable immune checkpoint inhibitors include antibodies against CTLA-1 (Ipilimumab), PD-1 (Nivolumab), or PD-L1 (Atezolizumab, Durvalumab und Avelumab).

Another subject-matter of the invention relate to an isolated peptide for use in the therapy and/or prophylaxis of a disease, preferably a tumor disease, wherein said peptide is the first peptide of the pharmaceutical composition according to the invention.

Said first peptide according to the invention can also be used as an adjuvant, as most patients are vaccinated against measles virus thereby, allowing for activation of immune system.

Another subject-matter of the invention relate to an isolated peptide for use in the therapy and/or prophylaxis of a disease, preferably a tumor disease, wherein said peptide is the second peptide of the pharmaceutical composition according to the invention.

The term "isolated" means that the peptide is removed from its original environment e.g., the natural environment, if it is naturally occurring. For example, a naturally- occurring peptide present in a living animal is not isolated, but the same peptide, separated from some or all of the coexisting materials in the natural system, is isolated. In an aspect, such peptide is part of the pharmaceutical composition and is not part of its natural environment. An isolated peptide also includes a synthetic peptide which is generated by means of peptide synthesis technologies.

The features, characteristics and advantages of the pharmaceutical composition according to the invention likewise apply to the afore-mentioned peptides according to the invention.

Another subject-matter of the invention relates to a nucleic acid encoding any of the afore-mentioned peptides according to the invention, and to an expression vector capable of expressing said nucleic acid.

As used herein, reference to a nucleic acid includes both single stranded and double stranded nucleic acid.

The features, characteristics and advantages of the pharmaceutical composition according to the invention likewise apply to the afore-mentioned nucleic acid and expression vector according to the invention.

Another subject-matter of the invention relates to a recombinant host cell comprising the peptide according to the invention, and/or the nucleic acid according the invention, and/or the expression vector according to the invention, wherein said host cell is preferably an antigen presenting cell, further preferably a dendritic cell. "Host cell" also includes chimeric antigen receptor T-cells.

The features, characteristics and advantages of the pharmaceutical composition according to the invention likewise apply to the afore-mentioned host cell according to the invention.

Another subject-matter of the invention relates to an antibody and/or T cell receptor, and/or chimeric antigen receptor T cells which specifically recognize the peptide according to the invention.

The features, characteristics and advantages of the pharmaceutical composition according to the invention likewise apply to the afore-mentioned antibody and/or T cell receptor and/or chimeric antigen receptor T cells according to the invention.

Another subject-matter of the invention relates to expression vectors, viruses, including lentiviruses, that can code for said afore-mentioned T cell receptors recognizing the peptides according to the invention.

Another subject-matter of the invention is a method for the treatment and/or the prophylaxis of a disease, preferably a tumor disease, further preferably a glioblastoma, comprising the step of administering to a patient in need, preferably to a patient with an HLA type HLA-A*24, further preferably with an HLA type HLA-A*24:03, of an therapeutically active amount of the pharmaceutical composition of the invention, and/or of the peptide of the invention, and/or of the antibody and/or T cell receptor of the invention.

The features, characteristics and advantages of the pharmaceutical composition according to the invention likewise apply to the afore-mentioned method according to the invention.

In an embodiment of the method according to the invention the administration of the pharmaceutical composition occurs after said patient has been treated with an oncolytic Measles vaccination virus, and preferably is followed by the additional administration of an immune checkpoint inhibitor.

Another subject-matter of the invention relates to a method for the treatment of a disease, preferably a tumor disease, further preferably a glioblastoma, characterized by a treatment sequence of chemotherapy, virotherapy, and radiotherapy (CT-VT-RT). Preferable chemotherapeutics include nitrosourea, such as temozolomide (TMZ), lomustine. Preferable virotherapeutics include oncolytic viruses, such as measles viruses (MeV). A preferably radiotherapy includes γ-irradiation, e.g. with doses of 0.5, 1, 2,3 or 4 Gy.

As the inventors were able to realize said treatment regimen or sequence, respectively, exhibits synergistic effects. This regimen can be further enhanced by peptide vaccinations with the newly identified and treatment-induced peptides according to the invention, potentially in combination with checkpoint inhibitors.

The features, characteristics and advantages of the pharmaceutical composition according to the invention likewise apply to the afore-mentioned method according to the invention.

It shall be understood that the features mentioned above and those to be mentioned in the following cannot only be used in the combination indicated in each case but also in other combinations or in isolated positions without departing from the scope of the present invention.

The invention is now explained in more details by means of embodiments which result in further characteristics, features and advantages. The embodiments are purely illustrative and do not restrict the scope of the invention. Features which are described in connection with a specific embodiment are isolated features of the invention in its general form and are not only features in the specific technical context.

Reference is made to the enclosed figures which show the following:

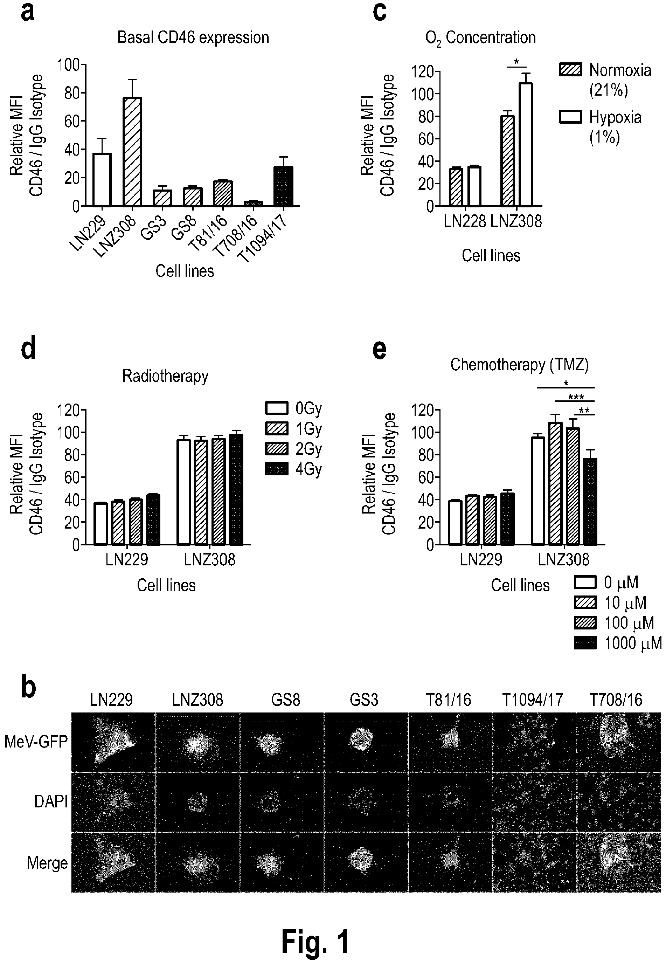

- Fig. 1:: MeV receptor expression and infectivity in glioma cells. a, Basal level of CD46 receptor expression in glioma cells, glioma stem-like cells, and primary glioblastoma cells determined via flow cytometry and depicted as relative MFI compared to isotype control. b, MeV infectivity observed via GFP-expression along with characteristic syncytial morphology as giant multinuclear aggregates visualized by fluorescence microscopy. Scale bar = 20 µm. c, Significant increase in CD46 surface expression on LNZ308 cells subjected to hypoxia with no effect of hypoxia in LN229 cells. d, No change in CD46 expression after γ-irradiation of LN229 or LNZ308 cells. e, Low dose (10 µM, 100 µM) TMZ increases CD46 surface expression while high dose of TMZ (1,000 µM) impairs CD46 expression in LNZ308 cells. Data expressed as Mean ± SEM Multiple t-test with Holm-Sidak post hoc test in c and two-way ANOVA with Tukey's multiple comparison test in e; *, p < 0.05; **, p < 0.01; ***, p < 0.001; ****, p < 0.0001. Data expressed as Mean ± SEM, n = 3 independent experiments.

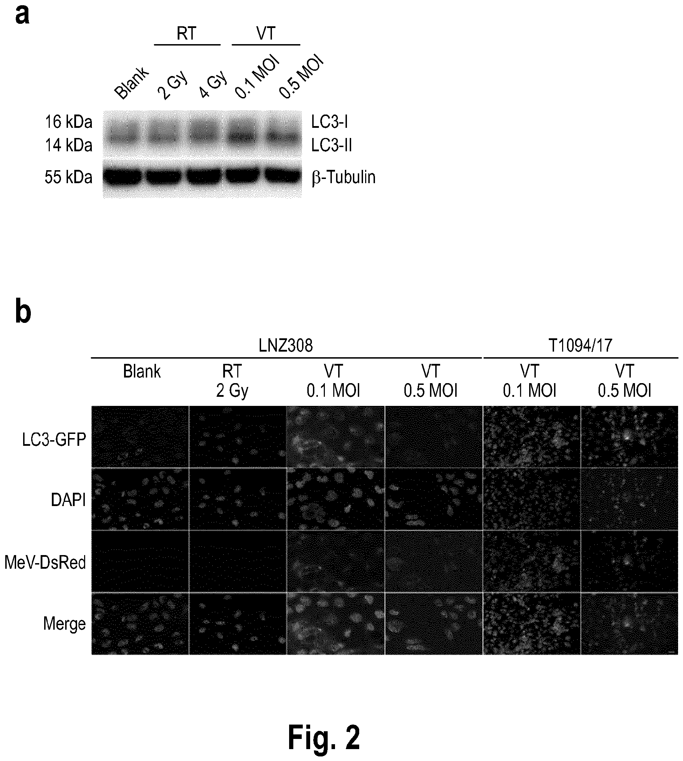

- Fig. 2:: Induction of autophagy upon MeV infection. a, Immunoblot analyses reveals conversion of LC3-I to LC3-II (upper panel) upon MeV infection (VT) in contrast to radiotherapy (RT); β-tubulin served as loading control (lower panel). b, VT initiates autophagic flux as demonstrated by an increased GFP-tagged LC3 expression in LNZ308 and primary GBM T1094/17 cells while basal expression seen in Blank untreated cells and upon radiotherapy (RT).

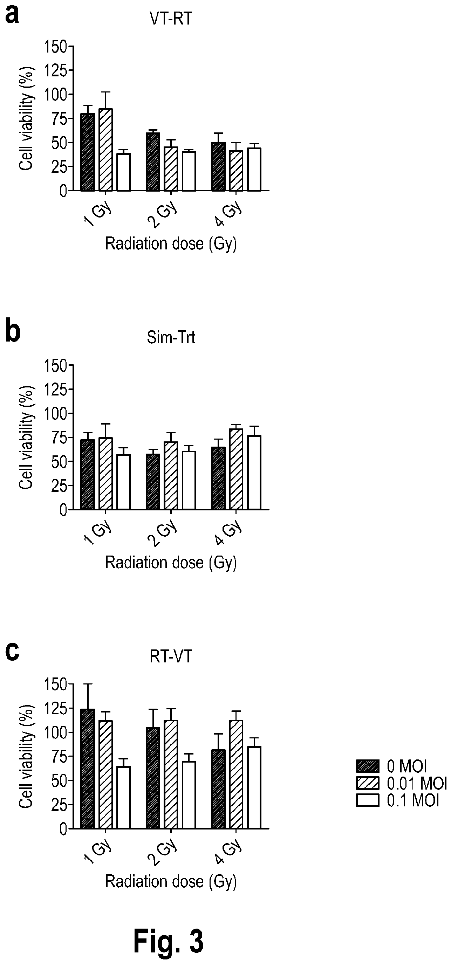

- Fig. 3:: Virotherapy followed by irradiation (VT-RT) is more efficient than other regimen in LN229. Cell viabilities assessed post sequential treatments with MeV and VT in a, VT-RT showing maximal synergistic potency in comparison to b, Simultaneous treatment (Sim-Trt) of MeV with RT or c, RT followed by VT, which shows less cytotoxic efficacy. Black, grey and white bars indicate viral doses in indicated multiplicity of infection (MOI) of 0, 0.01 or 0.1. Data expressed as Mean ± SEM, n=6 independent experiments.

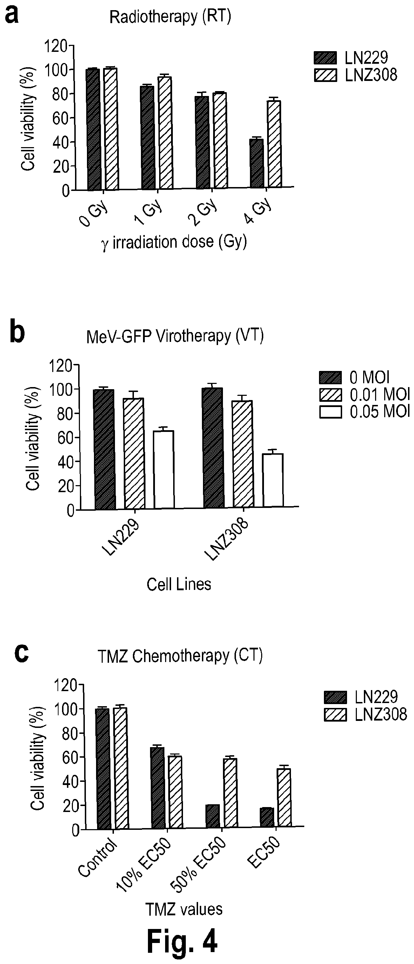

- Fig. 4:: Monotherapies post cytotoxic survival assay using temozolomide (TMZ) as an alkylating agent. LN229 and LNZ308 cells were treated with monotherapies of a, γ-irradiation (RT), b, MeV virotherapy (VT) or c, TMZ chemotherapy (CT). The cell viability post monotherapies were used to calculate predicted values of combinatorial treatment using fractional product method. Data expressed as Mean ± SEM, n=9 independent experiments.

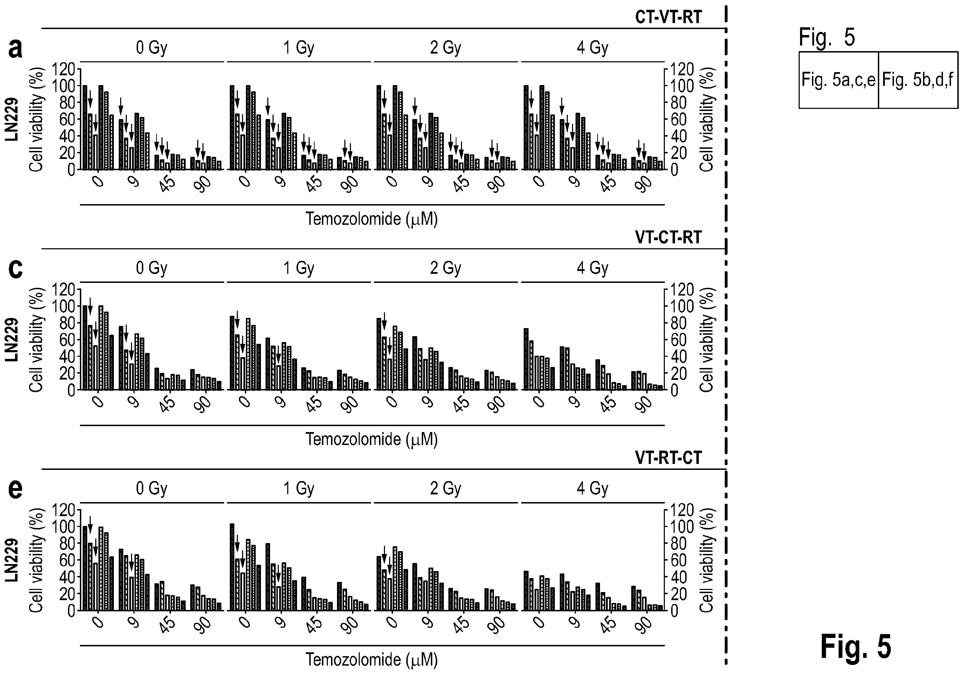

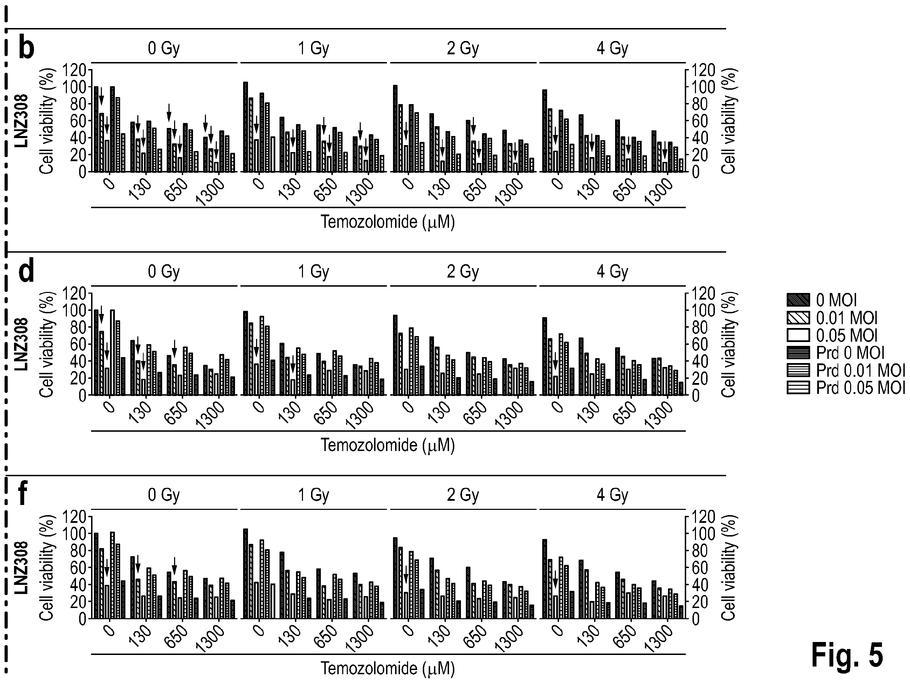

- Fig. 5:: CT-VT-RT exhibited synergy (TMZ as chemotherapeutic agent). CT-VT-RT elicits synergy in a, LN229 and b, LNZ308 cells. Non-synergistic regimen VT-CT-RT showed poor combinatorial efficacy in c, LN229 and d, LNZ308 cells. Similarly, regimen VT-RT-CT exhibited poor combinatorial efficacy in e, LN229 and f, in LNZ308. Red arrows show synergy realized in observed cell viability when compared to predictive values of cotreatments. Black, grey, and white bars indicate observed values at indicated MOI of VT. Orange, brown bars indicate calculated predictive values from individual monotherapies (RT, VT, CT). Abbreviations: Prd, predicted. Data expressed as Mean ± SEM, n = 9 independent experiments.

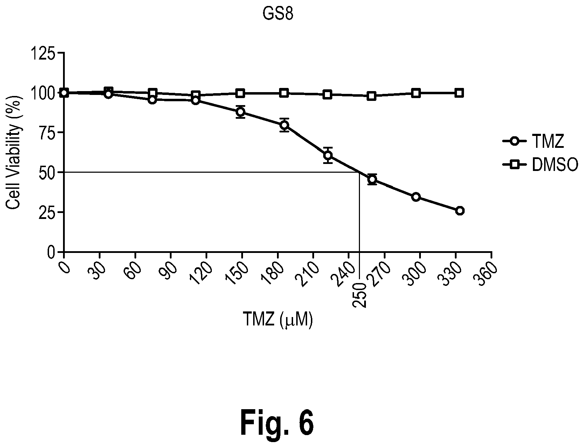

- Fig. 6:: Determination of EC₅₀ value for TMZ in GS8 cells. The EC₅₀ for GS8 was identified to be 250 µM, carried out using respective DMSO controls. TMZ and DMSO are depicted by blue and red dose-response curves respectively. Data expressed as Mean ± SEM, n = 9 independent experiments.

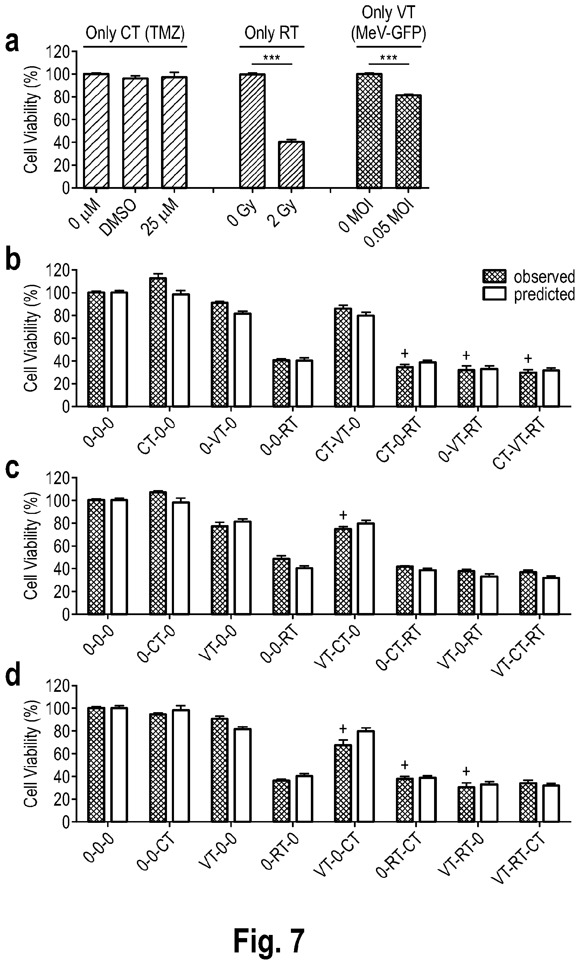

- Fig. 7:: Cytotoxic survival assay in GS8 using TMZ as alkylating agent. a, Monotherapies with TMZ (CT), γ-irradiation (RT), and MeV (VT). b - d, Black bars indicate observed values and white bars indicate calculated predicted values in the triple regimen. Triple regimen carried out with 0 indicate absence of treatment (0 Gy, 0 MOI or 0 µM) while CT = 25 µM, VT = 0.05 MOI, RT = 2 Gy as performed chronologically in that regimen. For example, in CT-0-RT, 0 MOI, i.e. no virus was used for VT, while TMZ concentration in CT was 25 µM, and RT was done with 2 Gy. Similarly, position of treatment is indicated in accordance with chronology within each regimen, e.g. VT-x-x indicates a regimen initiated with VT, while x-VT-x indicates that virotherapy came second 12 h after the first treatment. Hereby, we can visualize all observed monotherapies, dual therapies, and triple therapies with '+' indicating synergy when comparing observed vs. predicted cytotoxicity. b, CT-VT-RT is the only regimen to elicit synergy as a triple regimen, while d, the sequence VT-RT-CT revealed synergy in dual therapies, but not as a triple regimen. Data expressed as Mean ± SEM, n = 9 independent experiments.

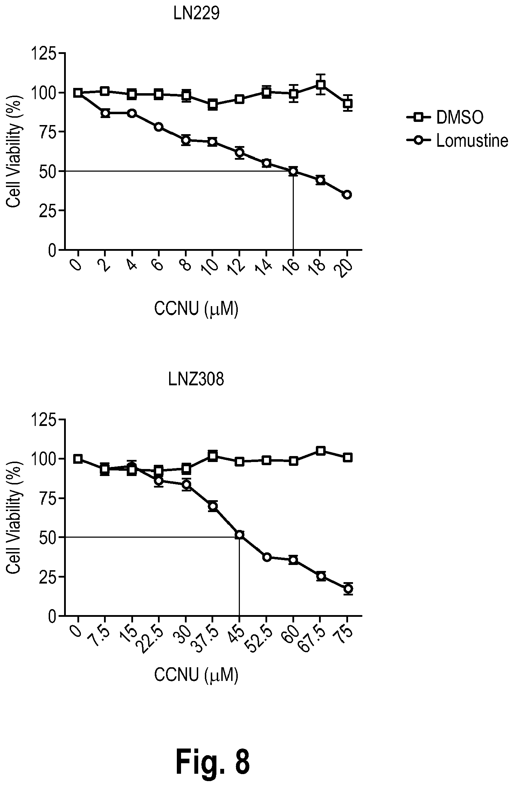

- Fig. 8:: Determination of EC₅₀ value for CCNU in LN229 and LNZ308 cells. Cell viability of LN229 or LNZ308 cells after incubation with different concentrations of CCNU or DMSO (control). The EC₅₀ for LN229 and LNZ308 were identified to be 16 µM and 45 µM), respectively. Blue line indicates cell viability post CCNU, while red line depicts viability under DMSO control treatment. Data expressed as Mean ± SEM, n = 6 independent experiments.

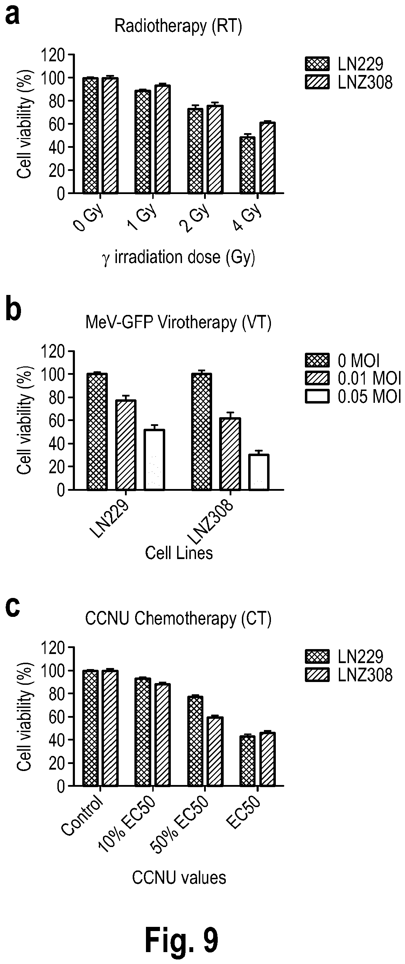

- Fig. 9:: Cytotoxic survival assay using lomustine (CCNU) as alkylating agent. LN229 and LNZ308 cells were treated with monotherapies of a, γ- irradiation (RT), b, MeV virotherapy (VT), and c, CCNU chemotherapy (CT). The cell viability post monotherapies were used to calculate predicted values of combinatorial treatment using fractional product method. Data expressed as Mean ± SEM, n = 9 independent experiments.

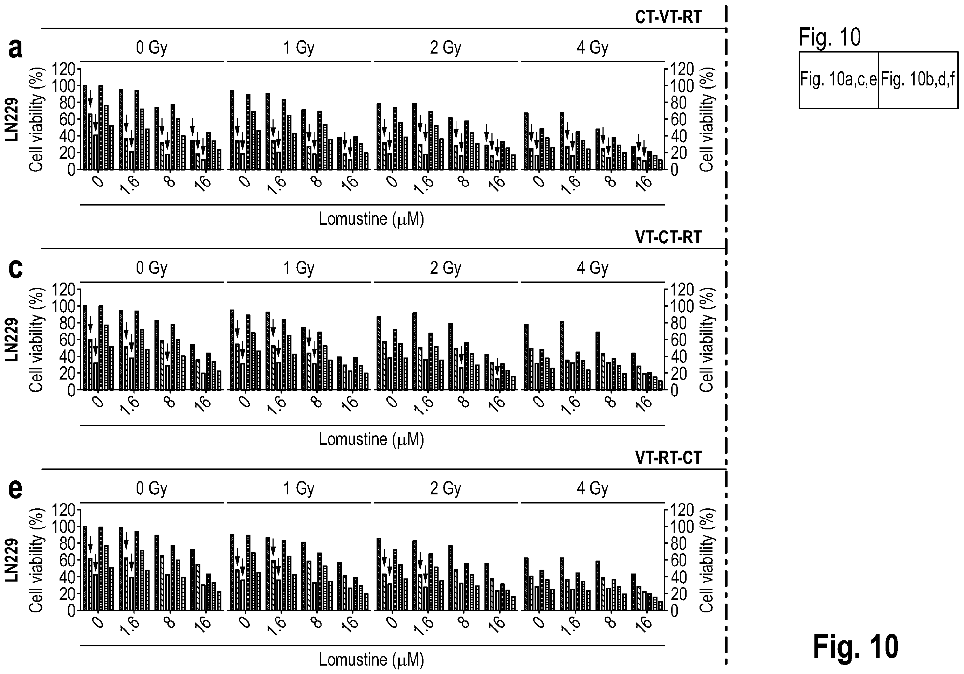

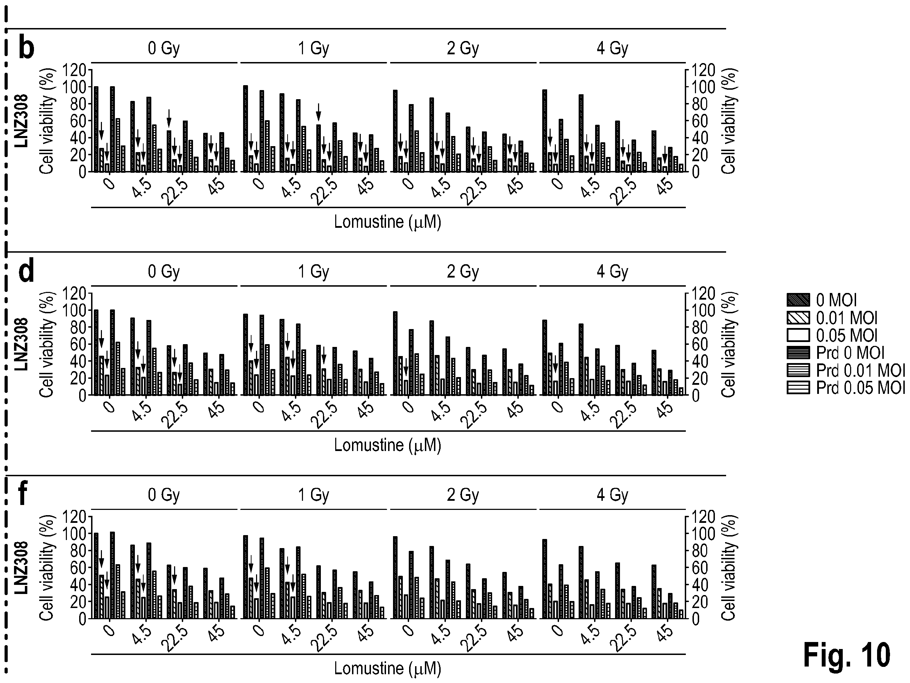

- Fig. 10:: CT-VT-RT exhibited synergy (CCNU as chemotherapeutic agent). CT-VT-RT is synergistic in a, LN229 and b, LNZ308 cells. VT-CT-RT showed poor combinatorial efficacy in c, LN229 and d, LNZ308 cells. Similarly, regimen VT-RT-CT exhibited poor combinatorial efficacy in e, LN229 and f, in LNZ308. Red arrows show synergy realized in observed cell viability when compared to predictive values of co- treatments. Black, grey, and white bars indicate observed values. Orange, brown bars indicate calculated predictive values from individual monotherapies (RT, VT, CT). Abbreviations: Prd, predicted. Data expressed as Mean ± SEM, n = 9 independent experiments.

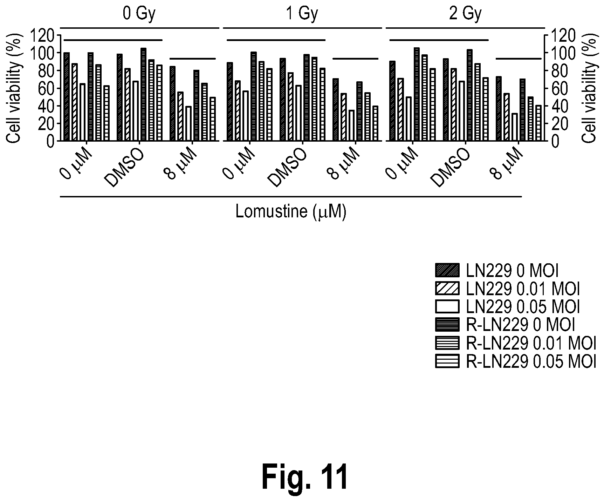

- Fig. 11:: Synergistic triple regimen CT-VT-RT using CCNU as alkylating agent in TMZ-resistant cells (R-LN229). Black, grey, and white bars indicate cell viability post CT-VT-RT treatment in parental LN229 cells. Orange and brown bars indicate cell viability post CT-VT-RT treatment in temozolomide-resistant R-LN229 cells. Addition of CCNU rescued the resistant effect (red lines) in synergistic regimen as opposed to aggressive proliferative effect (blue lines) in absence of CCNU (0 µM or DMSO) with increasing doses of γ-irradiation. Abbreviations: R-LN229, temozolomide-resistant LN229 cells; Prd, predicted. Data expressed as Mean ± SEM, n = 9 independent experiments.

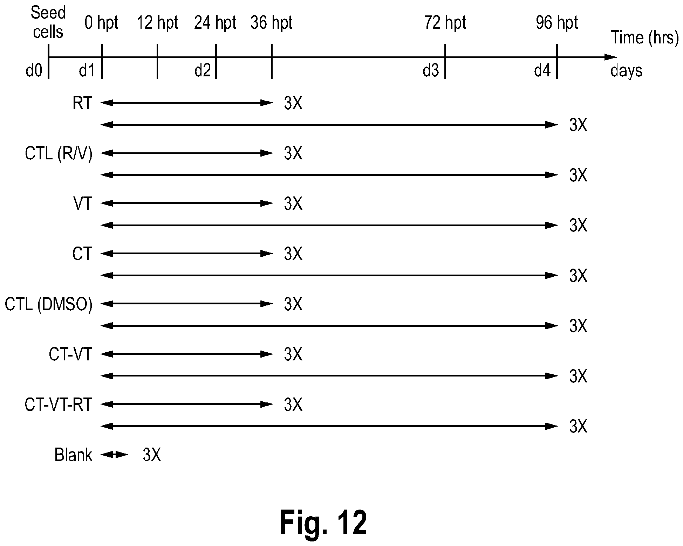

- Fig. 12:: Schematic representation of treatments and time points of samples considered for RNASeq. LNZ308 cells were treated with monotherapies (RT - 2 Gy, CT - 130 µM TMZ, VT - 0.05 MOI), double regimen (CT-VT, 130 µM TMZ - 0.05 MOI) and synergistic triple regimen (CT-VT-RT, 130 µM TMZ - 0.05 MOI - 2 Gy) along with CTL (DMSO) as control for CT initiated regimens and CTL (R/V) serving as control for RT and VT regimens. Blank (0 hpt) was used as basal control for expression. RNA and protein lysates were harvested at 0 hpt, 36 hpt, 72 hpt, and 96 hpt post respective treatments. Only time points marked in green were further processed for RNA sequencing (0 hpt, 36 hpt, and 96 hpt) as indicated with double-headed arrows. Samples at time-point 72 hpt (in blue) were collected, but solely used for validation of transcriptome data. "3X" indicate that all treatments were carried out in biological triplicates.

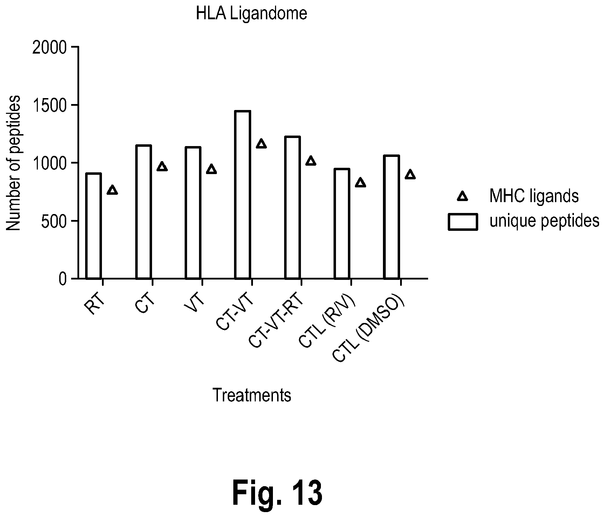

- Fig. 13:: Graph enumerating peptides and source proteins isolated from LNZ308 cells post individual treatments using HLA ligandomics. The samples were normalized using similar cell seeding density prior to treatment and area adjustment during measurement. Post-normalization, CT-VT showed maximal presentation of peptides with 1,430 peptides followed by CT-VT-RT with 1,222 peptides found.

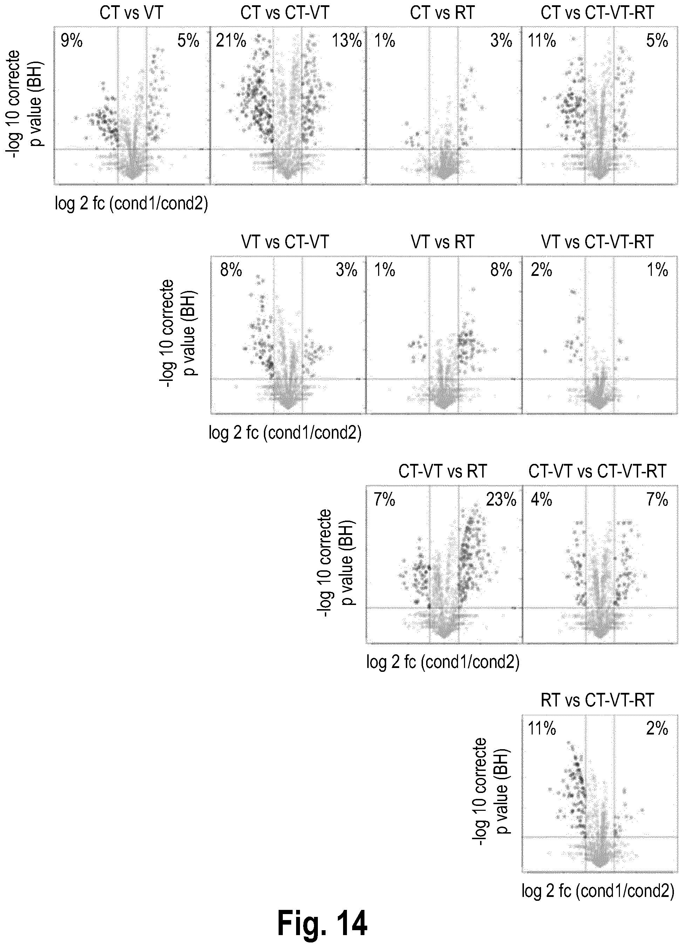

- Fig. 14:: Semi-quantitative volcano plot analysis visualizing modulations in HLA ligands presented on LNZ308 cells upon treatments. Each dot represents a HLA ligand with log2-fold changes with HLA ligand number indicated on the X-axis and the respective Benjamini-Hochberg corrected significance levels on the Y-axis. Significantly upregulated or downregulated HLA ligands (> 4-fold difference in number; P<0.01) are highlighted in red and blue respectively. The significantly modulated HLA ligand percentages are mentioned in the quadrants.

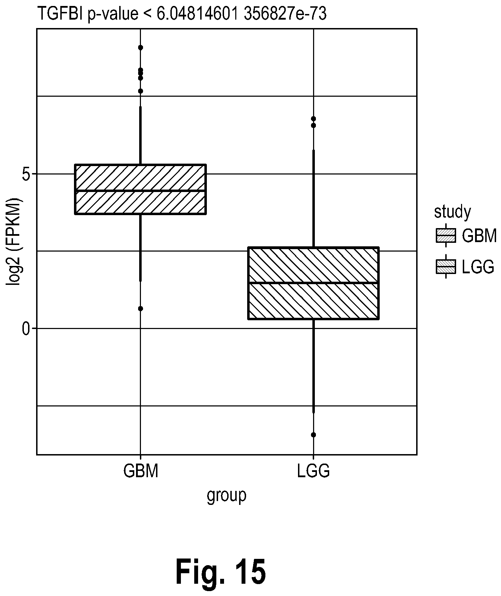

- Fig. 15:: Expression profile of TGFBI analyzed in TCGA dataset. TGFBI was significantly overexpressed in glioblastoma patients (GBM) in comparison to patients with low grade gliomas (LGG). The TGFBI expression represented in terms of log2 (FPKM). Abbreviations: TGFBI, TGF-β induced; GBM, glioblastoma; LGG, low grade glioma; FPKM, fragments per kilobase of transcript per Million mapped reads. P-value : 6.04×10⁻⁷³.

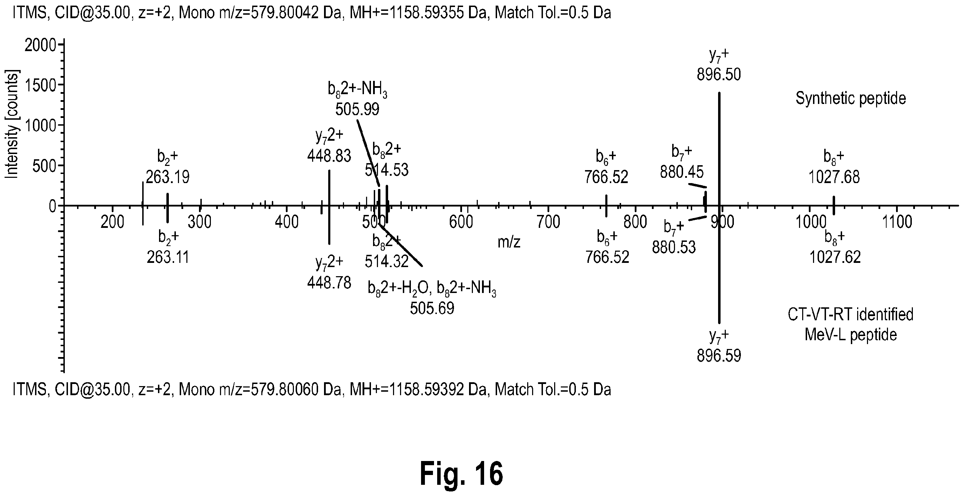

- Fig. 16:: MS-spectra match confirming identification of MeV-L peptide in CT-VT-RT treated LNZ308 cells. Mass spectrometry spectra match between synthesized peptide (upper axis, above 0) and CT-VT-RT identified MeV-L peptide (lower axis, below 0) from LNZ308 cells.

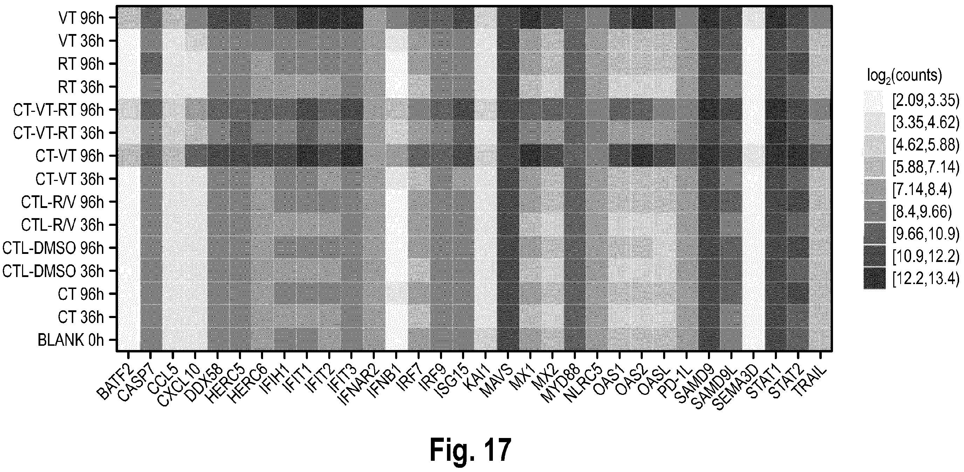

- Fig. 17:: Transcriptional signature heatmap of selected genes driving synergy post CT-VT-RT identified using RNASeq. The list of genes (X-axis) were identified as key factors in determining synergy and their transcriptional profile in all treatments at 36 and 96 hpt (Y-axis) represented as log2 counts (mRNA copy numbers) in a heatmap. Blank (0 hpt) served as the basal expression control for further analysis.

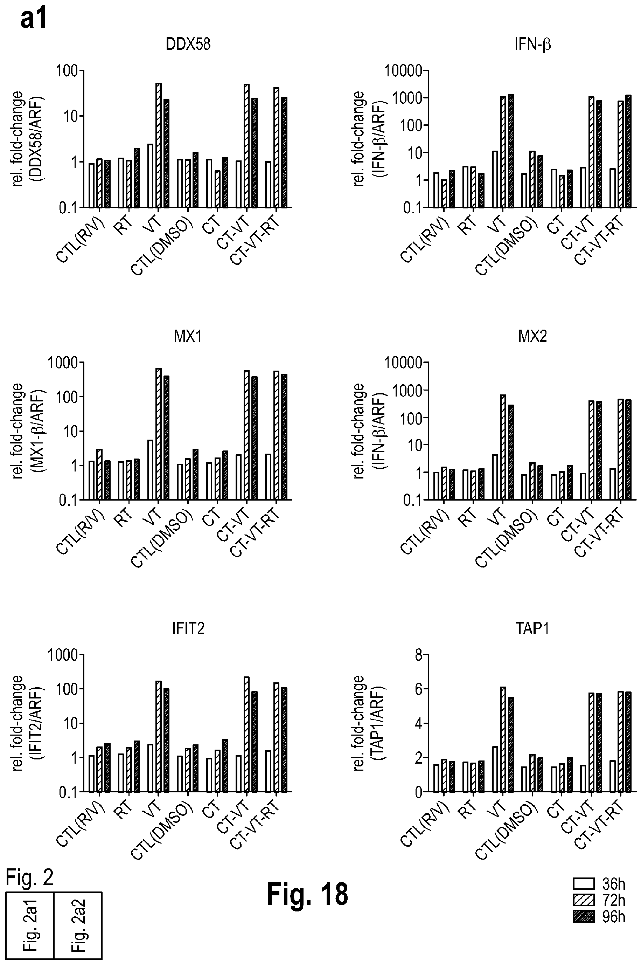

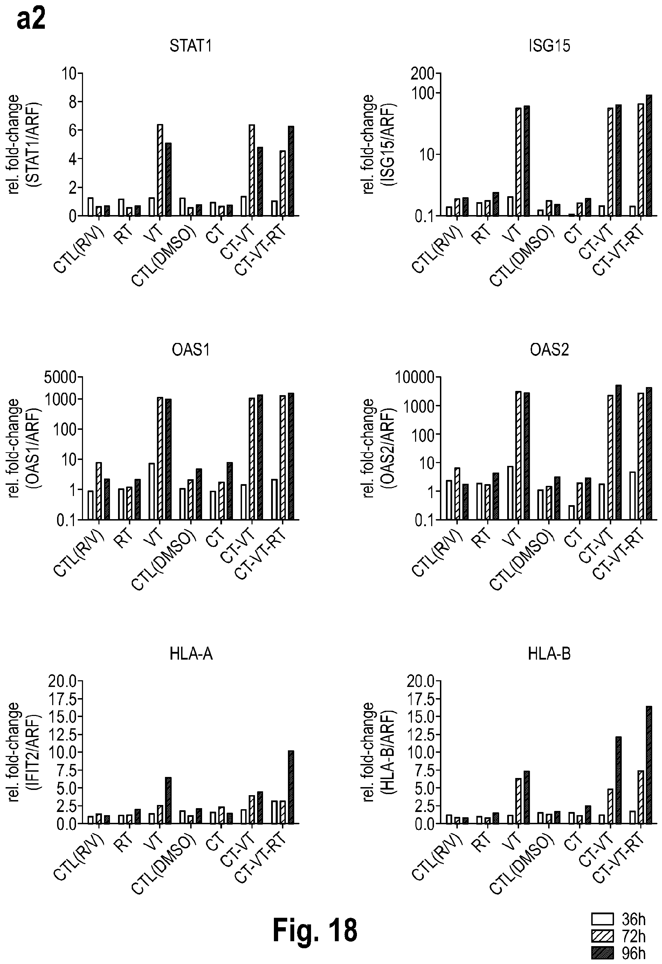

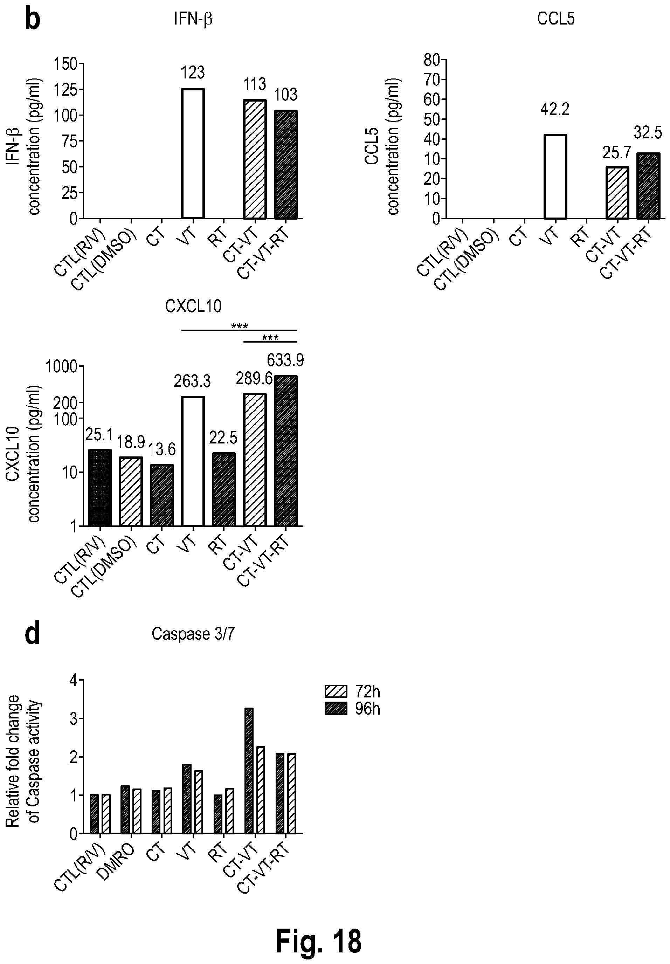

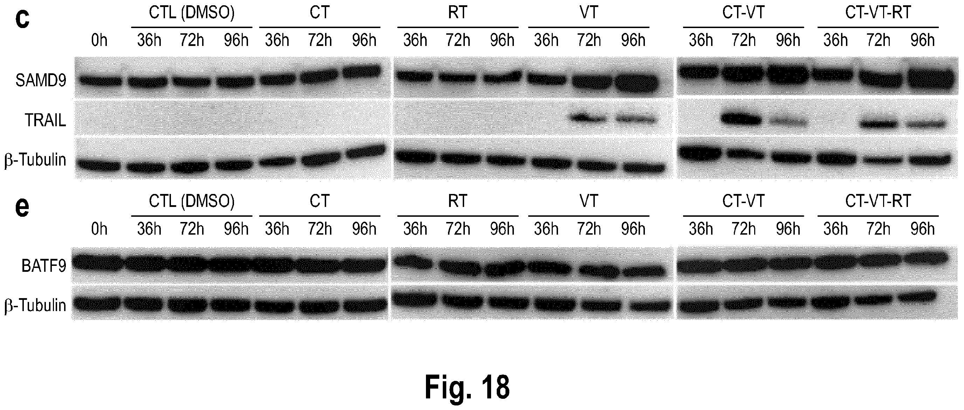

- Fig. 18:: Molecular profile in CT-VT-RT-treated LNZ308 cells. a, Fold change in mRNA expression levels of selected genes identified by RNASeq validated at sequential time points post treatment (36, 72, and 96 hpt) by qPCR. b, ELISA revealed production of IFN-β along with secretion of pro-inflammatory chemokines CCL5 and CXCL10 measured at 96 hpt. c, Immunoblot analysis revealed overexpression of SAMD9 (upper panel) in MeV-containing regimen and the induction of TRAIL (middle panel). β-tubulin (lower panel) served as loading control. d, Increased effector Caspase 3/7 activity detected in MeV-containing regimen over respective control, CTL (R/V) regimen. e, Immunoblot analysis showed no change in BATF2 protein expression (upper panel), β-tubulin (lower panel) served as loading control.

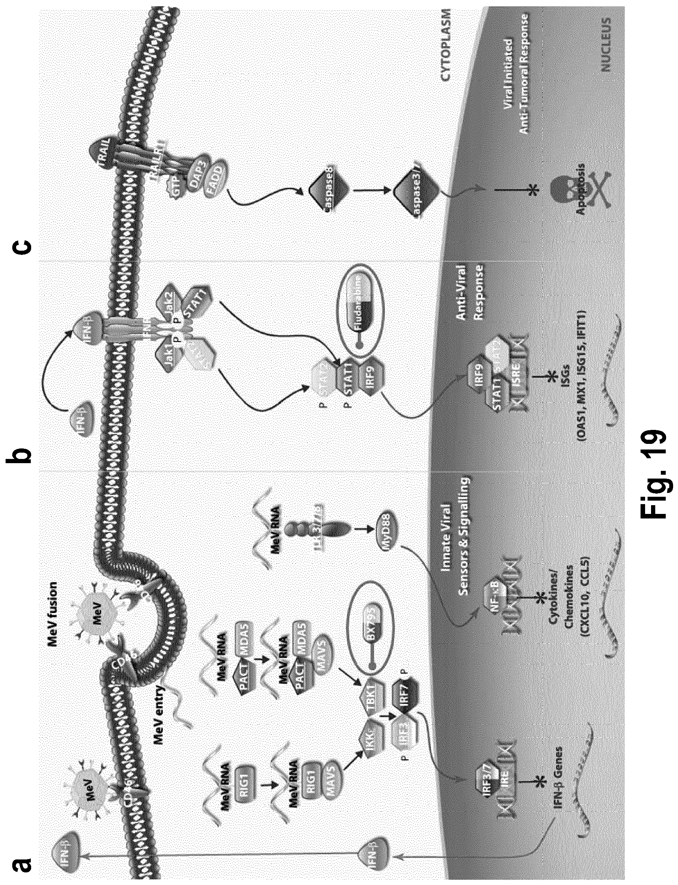

- Fig. 19:: Schematic overview of potential molecular drivers in CT-VT-RT based on RNA sequencing and validation data. a, MeV entry via receptordependent membrane fusion leads to insertion of RNP-associated viral genome, which is recognized by innate RLR signalling pathways (RIG-I, MDA5) that initiate a type I Interferon response through secretion of IFN-β along with proinflammatory chemokines. b, Activation of JAK-STAT signalling by IFN-β leads to transcription of ISGs along with maintenance of autocrine STAT1-signalling eventually triggering c, TRAIL-dependent apoptosis of glioma cells in addition to the oncolytic death cascade initiated by the virus. Red circles highlight molecular inhibitors BX795 and fludarabine; white font color, human molecules; black font color, viral molecules. Underlined molecules were not identified as significantly interacting genes in the RNASeq.

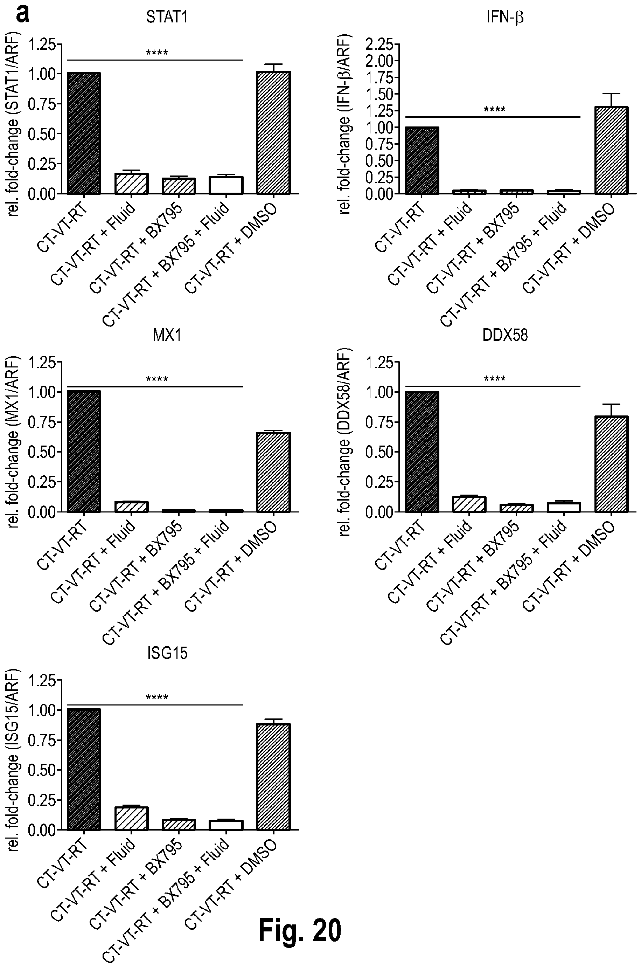

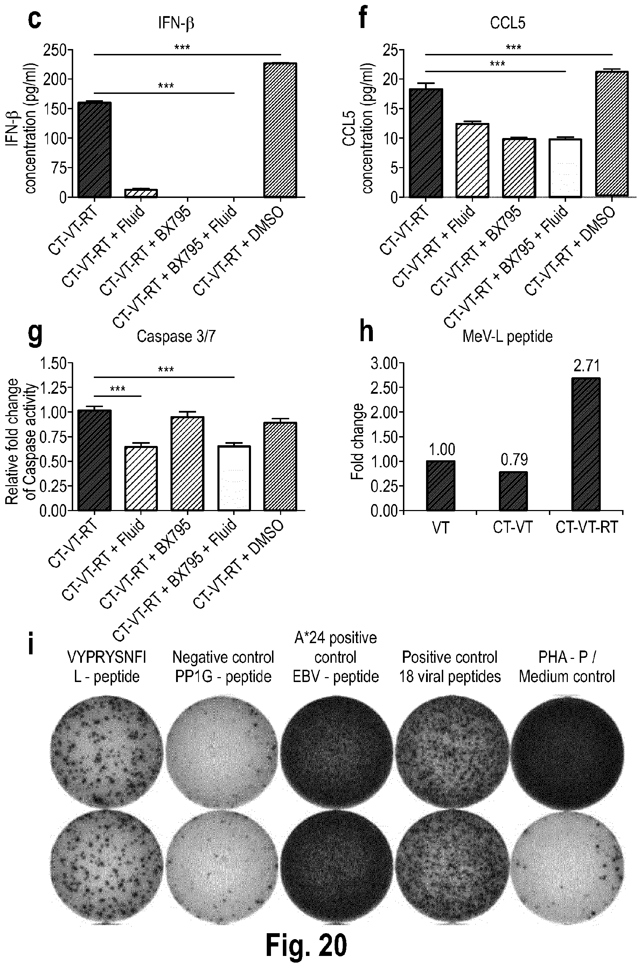

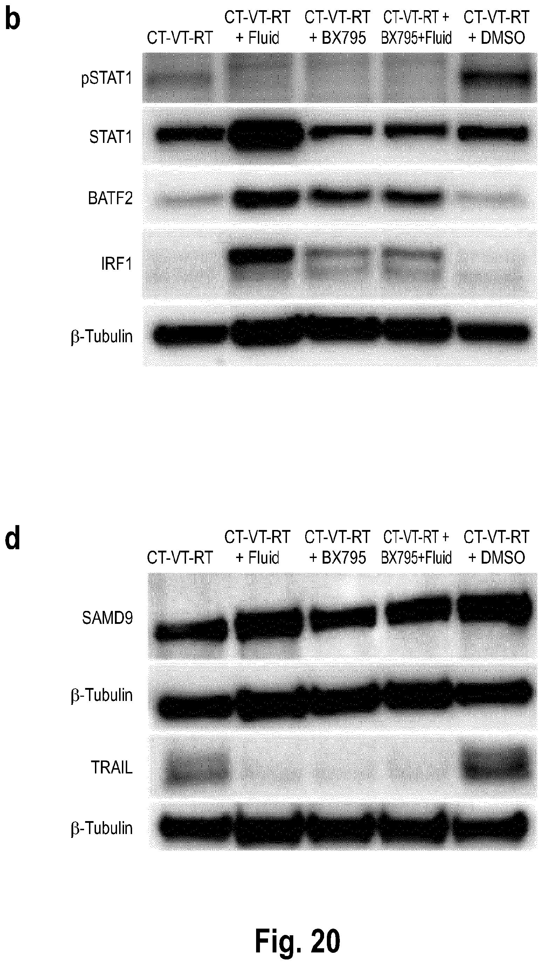

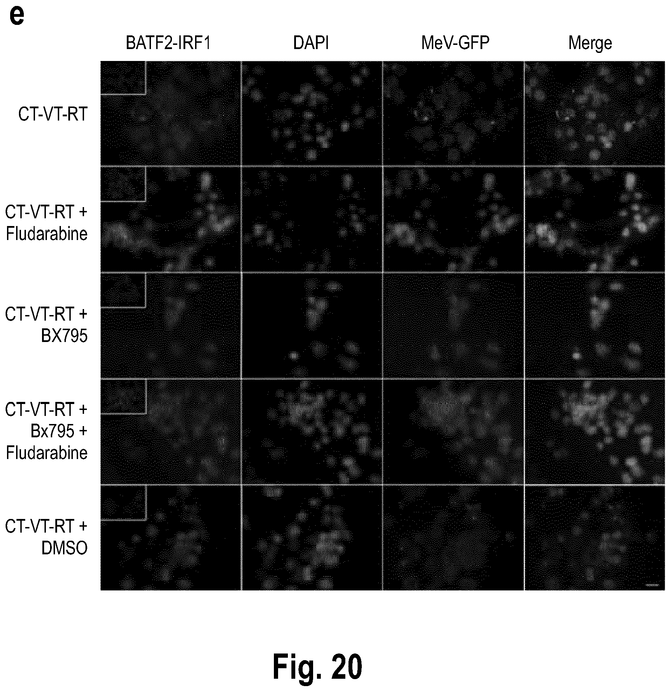

- Fig. 20:: Selective inhibition of canonical JAK-STAT signaling initiates alternative antiviral signaling network following immune-stimulatory CT-VT-RT treatment. a, Transcriptional profiles determined via qPCR revealing downregulation of IFN-β and STAT1 upon inhibitor treatment further also affecting downstream ISGs (MX1, ISG15) and DDX58. b, Immunoblot analysis after CT-VT-RT regimen with or without signalling cascade inhibitors monitoring levels of STAT1, phosphorylated STAT1 along with BATF2, IRF1, and β-tubulin as loading control as indicated. c, Loss of IFN-β production upon inhibitor treatment determined by ELISA. d, Immunoblot analysis post CT-VT-RT regimen with inhibitors. No change in SAMD9 expression (upper panel), but abrogation of TRAIL expression (lower panel) is shown upon inhibition; β-tubulin served as loading control. e, Proximity ligation assay reveals potential molecular interaction of BATF2 with IRF1 observed through increased red amplification signal in cells treated with CT-VT-RT plus fludarabine. Basal signal was observed in CT-VT-RT and CT-VT-RT plus DMSO alongside IgGs (Inset) in all treatment regimen. Scale bar = 20 µm. f, Decreased, but detectable CCL5 expression in samples treated with CT-VT-RT plus molecular inhibitors determined via ELISA. g, Effector Caspase 3/7 activity in samples treated with CT-VT-RT plus molecular inhibitors indicate alternative host machinery initiating apoptosis. h, CT-VT-RT increased presentation of MeV L-peptide, VYPRYSNFI on HLA-A*24 with a 2.71-fold increase over virotherapy alone. i, VYPRYSNFI stimulated healthy donor PBMCs to secrete IFNγ indicating immunogenic potential of the peptide to elicit CD8+ T cell responses in ELISpot analyses with appropriate positive and negative controls. One-way ANOVA with Dunnett's multiple comparison test; *, p < 0.05; **, p < 0.01; ***, p < 0.001; ****, p < 0.0001. Abbreviations: Flud, fludarabine.

### EXAMPLES

The following examples are presented to illustrate the present invention and to assist one of ordinary skill in making and using the same. The examples are not intended in any way to otherwise limit the scope of the invention.

### 1. Material and Methods

### Cell lines & primary tissue

The human long term glioma cell lines LN229, LNZ308, and the human embryonic kidney cell line 293T were obtained from American type culture collection (ATCC, Manassas, USA) and cultured in Dulbecco's Modified Eagle Medium (Thermo Fisher Scientific, Waltham, USA) supplemented with 10% fetal bovine serum and 50 µg/ml gentamycin (Thermo Fisher Scientific). The measles virus producer cell line 293-3-46 was a kind gift by Dr. Roberto Cattaneo and Dr. Stephen Russell and African green monkey kidney Vero B4 cells were obtained from Leibniz Institute DSMZ - German Collection of Microorganisms and Cell Cultures GmbH (DSMZ, Braunschweig, Germany) and cultured as described. The glioma stem-like (GS) cell lines, GS3 and GS8 were kindly provided by Katrin Lamszus. The primary tumor cell lines T81/16, T708/16, T1094/17 were harvested from fresh glioblastoma tissue obtained from patients undergoing surgery at the Department of Neurosurgery, University Hospital Tübingen (Ethical approval number: 456/2009B02) and cultured similar to GS cells in Neurobasal medium (Thermo Fisher Scientific) supplemented with 20 ng/ml recombinant human fibroblast growth factor (Peprotech, New Jersey, USA), 20 ng/ml recombinant human epidermal growth factor (Peprotech), 2 mM L-glutamine (Thermo Fisher Scientific), 10% B-27 supplement (Thermo Fisher Scientific) and 50 µg/ml gentamycin (Thermo Fisher Scientific). Temozolomide (TMZ) resistant cell line R-LN229 were generated by repetitive exposure of parental LN229 to TMZ, as previously described.

### Flow cytometry for basal CD46 receptor expression

Cells were stained with 0.25 µg antibody (either CD46-PE (8E2) or IgG1k-PE; Thermo Fisher Scientific) and receptor expression measured along with viability dye, 7-aminoactinomycin D (7-AAD; Thermo Fisher Scientific) in MACSQuant Analyzer 10 (Miltenyi Biotec, Bergisch Gladbach, Germany). The ratio of mean fluorescence intensities (MFI) of PE-conjugated CD46 vs isotype control were calculated and graphs generated using GraphPad Prism 6 software (GraphPad Software, La Jolla, USA).

### CD46 receptor expression after treatments in vitro

LN229 and LNZ308 cells were treated with TMZ (0 µM, 10 µM, 100 µM or 1,000 µM), γ-irradiation (0 Gy, 1 Gy, 2 Gy, or 4 Gy), and under different oxygen conditions (21% O₂ (normoxia) or 1% O₂ (hypoxia)) for 48 h. Then, levels of CD46 receptor expression were analysed via flow cytometry as described above along with viability staining using 7-aminoactinomycin D (7-AAD, Thermo Fisher Scientific) as described above.

### Measles Virus production

The recombinant Edmonston strain of measles virus (NSe) encoding green fluorescent protein (GFP) or the rapidly maturing variant of Discosoma sp. red fluorescent protein (DsRed) were rescued as described previously. The viruses were propagated in Vero cells and titers determined by 50% tissue culture infective dose titration (TCID50) by Kärber method.

### Fluorescence microscopy

Cells were seeded on glass chamber slides coated with poly-L-lysine (Sigma Aldrich, St. Louis, USA) alone for adherent cells or along with laminin (Sigma Aldrich) for suspension cultures one day before infection with MeV-Nse-GFP at 1 multiplicity of infection (MOI) for 72 h at 37°C. Cells were fixed with 4% paraformaldehyde followed by nuclear staining with 4',6-diamidino-2-phenylindole (DAPI; Thermo Fisher Scientific). Images were captured with Zeiss microscope using Apotome (Carl Zeiss, Oberkochen, Germany), and image analysis carried out with AxioVision software (Carl Zeiss).

### Acute cytotoxicity assay

LN229 and LNZ308 were seeded as 5,000 cells per well in 96 well plates one day before treatment initiation. All treatments were carried out in serum-free DMEM along with their respective controls, and cell viability measured at 72 h post initial treatment (hpt) using Cell titer blue solution (Promega, Madison, USA) as per manufacturer's instructions. The measured values were converted to percentage cell viability by normalizing each treatment to its respective control within the individual regimen.

### Clonogenic survival assay

LN229 and LNZ308 were seeded as 1,000 cells per well in 6 well plates one day before treatment initiation. All treatments were carried out in serum-free DMEM with their respective controls and cells allowed to form clones defined as individual population consisting of >50 cells. The cells were stained with 0.5% crystal violet solution (dissolved in 20% methanol) and clones counted manually by three independent blinded scientists (SR, DC, RS).

### Cytotoxic survival assay (CSA)

The inventors designed a cell viability assay to elicit and determine the oncolytic cascade effect initiated by MeV in combination with cytotoxic agents such as TMZ. Herein, measurement of cytotoxicity associated with recurrent ineffective mismatch repair or lysis of MeV-induced syncytia require longer readout periods of 144 hpt as opposed to a classical 72 hpt acute cytotoxicity assay.

LN229, R-LN229, and LNZ308 cells were seeded as 5,000 cells per well in 96 well plates one day before treatment initiation with γ-irradiation (RT), alkylating chemotherapy (CT), or MeV virotherapy (VT), alone, or sequentially as combinatorial regimens in serum-free DMEM at the indicated concentrations or doses. The cell viability was measured at 144 hpt using Cell titer blue solution (Promega) as per manufacturer's instructions, and measured values were converted to percentage cell viability by normalizing each treatment to its respective control within the individual regimens. The predicted values of combinations used to determine synergy were calculated from the individual monotherapies; RT, CT and VT by fractional product method defined as the product of the viable fractions after treatment with all agents alone. If the observed value of the co-treatment is less than that of the calculated predictive value, the combination of agents was deemed to be synergistic and not only additive. This is accordance with an alternative method of synergy calculation using the coefficient of drug interaction without conversion to ratios.

GS8 cells were seeded as 50,000 cells per 35 mm dish one day before treatment initiation, and all treatments performed in serum-free neurobasal medium. The measurement of cell viability was performed in triplicates with 5,000 cells at 144 hpt using Cell titer blue solution (Promega) as described previously. The graphs were generated using GraphPad Prism 6 with percentage cell viability post treatments, and synergistic regimens were identified as described above.

### LC3-GFP puncta assay

Cells were treated with different treatment regimens (Blank, RT, VT) in serum-free medium followed by serum supplementation. Cells were stained for LC3-GFP 12 hpt along with nuclear Hoechst 33342 using CYTO-ID Autophagy detection kit 2.0 (Enzo Life Sciences, Lausen, Switzerland) as per manufacturer's instructions. Images were captured with Zeiss microscope using Apotome (Carl Zeiss) and image analysis carried out with Axiovision software (Carl Zeiss).

### Immunoblotting

Cells of interest were lysed in lysis buffer containing 25 mM Tris hydrochloric acid (Tris-HCI), 120 mM sodium chloride (NaCI), 5 mM ethylenediaminetetraacetic acid (EDTA), 0.5% NP-40 and phosphatase inhibitor cocktails (Sigma Aldrich). Proteins were separated on 4-12% polyacrylamide gels (Thermo Fisher Scientific) followed by transfer to PVDF membranes (Thermo Fisher Scientific). The blots were blocked in Tris-buffered saline containing 5% skim milk (Becton Dickinson, New Jersey, USA) and 0.05% Tween 20 (Sigma Aldrich) for 1 h. Protein blots were probed with primary antibodies overnight at 4°C and, after thorough washing, for 1 h at room temperature with horseradish peroxidase-coupled secondary antibodies (Abcam, Cambridge, UK) the following day. Protein bands were visualized using Pierce chemiluminescent substrate solutions (Thermo Fisher Scientific), and image capture and analysis carried out with ChemiDoc MP imaging system (Bio-Rad Laboratories, Hercules, USA) using Image lab software (Bio-Rad Laboratories).

### Antibodies and reagents

Antibodies against SAMD9 (EPR13603), BATF2 (EPR10667), STAT1 -α (EPYR2154), STAT1 phospho-Y701 (M135) and IRF1 (EPR18301) were purchased from Abcam (Abcam) and used at a dilution of 1:1,000. Antibody against TRAIL (C92B9), LC3A (D50G8) and β-tubulin (9F3) were purchased from Cell Signalling (Cell Signalling, Dan-vers, USA) and used at a dilution of 1:1,000. Anti-rabbit and Anti-mouse horseradish peroxidase coupled secondary antibodies were purchased from Abcam and used at a concentration of 1:5,000. TBK1/IKKε inhibitor, BX795 was purchased from Invivogen (Invivogen, San Diego, USA) and used at a concentration of 10 µM. Fludarabine (Selleck-chem, Houston, USA) was used as a functional STAT1 inhibitor at a concentration of 100 µM.

### RNA sequencing (RNASeq) and analysis

LNZ308 cells were seeded as 3×10⁶ cells per dish and treated with monotherapies (RT, 2 Gy; CT, 130 µM; VT, 0.05 MOI), double regimen (CT-VT, 130 µM - 0.05 MOI), synergistic triple regimen (CT-VT-RT, 130 µM - 0.05 MOI - 2 Gy) along with DMSO as control for CT initiated regimens (CTL-DMSO), and controls for RT alone and VT alone regimens (CTL (R/V)) under serum-free conditions. Blank (0 hpt) served as basal control for expression. RNA was isolated at 36 hpt and 96 hpt using RNeasy Mini kit (Qiagen, Hilden, Germany) according to manufacturer's instructions and RNA quality was assessed with an Agilent 2100 Bioanalyzer (Agilent Technologies, Santa Clara, USA). Samples with high RNA integrity number (RIN > 8) were selected for library construction. Using the TruSeq Stranded RNA Sample Prep Kit (Illumina, San Diego, USA) and 400 ng of total RNA for each sequencing library, poly(A) selected single-read sequencing libraries (68 bp read length) were generated according to the manufacturer's instructions. All libraries were sequenced on an Illumina HiSeq2500 platform (Illumina) at a depth of 18-20 million read search. Library preparation along with sequencing procedures were performed by the same individual to minimize technical batch effects. Raw fastq files were pre-filtered using the chastity filter to remove reads that contain a "Y" flag. FastQC (Andrews S. (2010), FastQC: a quality control tool for high throughput sequence data. Available online at: http://www.bioinformatics.babraham.ac.uk/projects/fastqc) was used to determine quality of the resulting fastq files. Subsequently, adapter trimming/removal process was conducted with Cutadapt (https://pypi.python.org/pypi/cutadapt/), version 1.8.3). This step used the FastQC output to identify reads that showed a match to some typical overrepresented (Illumina) sequences/adapters. TopHat2 was used as aligner to map the quality controlled remaining reads to the human genome. Read counting to features (genes) in the genome was performed with HTSeq (http://www-huber.embl.de/users/anders/HTSeq/doc/count.html, version 0.6.0.). Counting was performed using "union" mode on the feature "gene_id", where each gene is considered here as the union of all its exon counts. The stranded option was also set to "stranded=no" to count features on both strands. For differential expression analysis the raw read count table provided by HTSeq was used into the R package DESeq2 (version 1.10.1). Adjusted p-values were used at an FDR (False Discovery Rate) < 0.05 to account for multiple hypothesis testing.

### Enzyme linked immunosorbent assay (ELISA)

LNZ308 cells were seeded as 3×10⁶ cells per dish and treated with different regimens as described above, supernatants were harvested after 96 hpt and concentrated using Amicon Ultra-15 Ultracel-3K centrifugal filters (Merck Millipore, Billerica, USA) with subsequent determination of total protein concentration using Bradford protein assay (Bio-Rad Laboratories). The respective chemokine/cytokine concentrations were determined enzymatically per microgram of total protein using the respective DuoSet ELISA kits according to manufacturer's instructions (R&D Systems, Minneapolis, USA).

### Quantitative Real-time PCR (qPCR)

RNA extractions were performed using the RNeasy mini kit (Qiagen) and reversely transcribed to cDNA using High-Capacity RNA-to-cDNA kit (Thermo Fisher Scientific) according to manufacturer's instructions. For each qPCR reaction, 20 ng of cDNA were amplified with custom designed primers (Supplementary Table 1) and qPCR Mastermix Plus for SYBR Green (Eurogentec, Liege, Belgium) using the 7500 Fast Real time PCR system (Thermo Fisher Scientific). Relative gene expression was determined using the ΔΔ-CT method versus the housekeeping gene ARF1.

Supplementary Table 1. List of primers used for qRT-PCR analysis. The forward and reverse primers used for validation of genes identified from RNASeq using qRT-PCR.

**PCR primer**

| Gene | Forward Primer (5'→3') | Reverse Primer (5'→3') |
|---|---|---|
| ARF1 | GACCACGATCCTCTACAAGC(SEQ ID NO: 45) | TCCCACACAGTGAAGCTGATG (SEQ ID NO: 46) |
| DDX58 | AGACAAAGATGAAGAGAGCAGGA (SEQ ID NO: 47) | GCTCGGACATTGCTGAAGAAG (SEQ ID NO: 48) |
| HLA-A | GAGTATTGGGACCAGGAGACA (SEQ ID NO: 49) | CGTCGCAGCCATACATTATCTG (SEQ ID NO: 50) |
| HLA-B | TGAGATGGGAGCCGTCTTC (SEQ ID NO: 51) | CTACACATCACAGCAGCGAC (SEQ ID NO: 52) |
| IFIH1 | CGGATATAAAGAATGTAACATTGTTATC (SEQ ID NO: 53) | ATGAGCATACTCCTCTGGTTTCA (SEQ ID NO: 54) |
| IFIT1 | CCTCCTTGGGTTCGTCTACA (SEQ ID NO: 55) | GGCTGATATCTGGGTGCCTA (SEQ ID NO: 56) |
| IFN-β | GTCTCCTCCAAATTGCTCTCC (SEQ ID NO: 57) | CAGTATTCAAGCCTCCCATTCA (SEQ ID NO: 58) |
| ISG15 | ATGGGCTGGGACCTGACG (SEQ ID NO: 59) | GCCGATCTTCTGGGTGATC (SEQ ID NO: 60) |
| MX1 | CGCTGGTGCTGAAACTGAAGA (SEQ ID NO: 61) | GCGATGGCATTCTGGGCTTTA (SEQ ID NO: 62) |
| MX2 | AGTTCAGAATGGAGCAGATGG (SEQ ID NO: 63) | ACCGAAGACTCATTACTGGGAA (SEQ ID NO: 64) |
| OAS1 | CACAGAACTACAGAGAGACTTC (SEQ ID NO: 65) | CAAGCATAGACCGTCAGGAG (SEQ ID NO: 66) |
| OAS2 | GACTTCTCCCAACCTGGATAATG (SEQ ID NO: 67) | CTGTCAATCTGCTCTAGGAAGC (SEQ ID NO: 68) |
| STAT1 | CAGAACAGAGAACACGAGACCA(SEQ ID NO: 69) | GTTCAGTGACATTCAGCAACTCTA (SEQ ID NO: 70) |
| TAP1 | AAAGACACTCAACCAGAAGGAG(SEQ ID NO: 71) | GCCCACCAATGTAGAGGATTC (SEQ ID NO: 72) |

### Isolation of HLA ligands

Cells subjected to treatment regimens as mentioned above (CSA) were harvested at 96 h, washed twice with cold phosphate buffered saline (PBS; Lonza Group, Basel, Switzerland), and stored frozen at -80°C with subsequent isolation of HLA class I molecules using standard immunoaffinity purification. In brief, cell pellets were lysed in 10 mM 3-[(3-Cholamidopropyl)-Dimethylammonio]-1-Propanesulfonate (CHAPS; Applichem, Gatersleben, Germany)/PBS (Lonza Group) containing protease inhibitor (Roche, Basel, Switzerland). HLA molecules were purified using the pan-HLA class I-specific monoclonal W6/32 Ab, which is covalently linked to CNBr-activated Sepharose (GE Healthcare, Little Chalfont, U.K.). Repeated addition of 0.2% trifluoroacetic acid (Merck Millipore) eluted HLA molecules and peptides. The peptides were isolated employing ultrafiltration with centrifugal filter units (Merck Millipore), extracted and desalted using ZipTip C18 pipette tips (Merck Millipore). Peptides were then eluted in 35 µL acetonitrile (Merck Millipore) / 0.1% trifluoroacetic acid, concentrated by vacuum centrifugation to 5 µL, and resuspended in 25 µL of 1% acetonitrile/0.05% trifluoroacetic acid. Finally, the peptide solutions were stored at -20°C until analysis by LC-MS/MS.

### Analysis of HLA Ligands by LC-MS/MS

Peptides were separated using reversed-phase liquid chromatography (nanoUHPLC, UltiMate 3000 RSLCnano; Dionex, Sunnyvale USA) and analysed in an online coupled LTQ Orbitrap XL hybrid mass spectrometer (Thermo Fisher Scientific). In five technical replicates volumes of 5 µL peptide solution (sample shares of 20%) were injected onto a 75 µm x 2 cm trapping column (Acclaim PepMap RSLC, Dionex) at 4 µL/min for 5.75 min. Peptide separation was performed at 50°C with a flow rate of 175 nL/min on a 50 µm x 50 cm separation column (Acclaim PepMap RSLC, Dionex) applying a gradient ranging from 2.4 to 32.0% of acetonitrile over the course of 140 min. Eluted peptides were ionized by nanospray ionization and analyzed in the mass spectrometer implementing a top 5 collision induced dissociation (CID) method generating fragment spectra for the five most abundant precursor ions in the survey scans. For HLA class I ligands, the mass range was limited to 400-650 m/z with charge states 2⁺ and 3⁺ selected for fragmentation and the resolution was set to 60,000.

### Database Search and Spectral Annotation

Data was processed against the LNZ308 proteome derived from RNA sequencing and the oncolytic measles virus proteome (UniProt) applying the SequestHT algorithm in the Proteome Discoverer 1.3 (Thermo Fisher Scientific) software. Precursor mass tolerance was set to 5 ppm, fragment mass tolerance was set to 0.5 Da, and oxidized methionine was allowed as a dynamic modification. Percolator assisted FDR calculation was set at a target value of q ≤ 0.05 (5% FDR). Peptide spectrum matches with q ≤ 0.05 were filtered according to additional orthogonal parameters to ensure spectral quality and validity. Peptide lengths were limited to 8-12 aa. HLA annotation was performed using NetMHCpan-3.0 based on the HLA class I typing of LNZ308.

### Analysis of LNZ308 ligandomes in different conditions

For label-free quantification of the relative HLA ligand abundances under the different conditions (CT, CT-VT, CT-VT-RT, VT and RT), the injected peptide amounts of paired samples were normalized and LC-MS/MS analysis was performed in five technical replicates for each sample. In brief, relative amounts of substance of paired samples were calculated from average precursor ion intensities determined in dose-finding mass spectrometry runs and adjusted accordingly by dilution. Relative quantification of HLA ligands was performed by calculating the area under the curve of the corresponding precursor extracted ion chromatograms using Proteome Discoverer 1.3. The ratios of the mean areas of the individual peptides in the five label-free quantification-MS runs of each sample were calculated and two-tailed t-tests were performed using an in-house Matlab script (v8.2, MathWorks, Natick, USA).

### Peptide synthesis

The automated peptide synthesizer Liberty Blue (CEM, Kamp-Lintfort, Germany) was used to synthesize peptides using 9-fluorenylmethyl-oxycarbonyl/tert-butyl (Fmoc/tBu) strategy. The identity and purity of peptides were validated by reversed-phase liquid chromatography (nanoUHPLC, UltiMate 3000 RSLCnano, Dionex) and on-line coupled LTQ Orbitrap XL hybrid mass spectrometer (Thermo Fisher Scientific) system. Synthesized peptide was used in the validation of LC-MS/MS identification as well as in functional experiments.

### T cell culture

Blood samples of healthy donors matched for HLA-A*24 serotype were kindly provided by the Institute for Clinical and Experimental Transfusion Medicine at the University Hospital Tübingen after obtaining written informed consent. Peripheral blood mononuclear cells (PBMCs) were isolated by standard Ficoll-Hypaque (Biocoll, Biochrom, Berlin, Germany) density gradient centrifugation. Cells were stored at -80°C in FCS (Sigma Aldrich) and 10% DMSO (Merck, Whitehouse Station, New Jersey, USA). After thawing, the cells were rested overnight prior to stimulation with culture conditions of 37°C and 7.5% CO₂ in humidified incubators.

### IFNγ ELISPOT Assay

The IFNγ ELISpot assay after 12 day stimulation was performed. Briefly, cells were stimulated 24 h after thawing with 1 µg/ml candidate MeV peptide or control peptides. IL-2 (R&D Systems) was added on days 2,5 and 7 with a final concentration of 20 U/ml or 1,000 U/ml for PBMCs of healthy donors or TILs, respectively. On day 12, cells were harvested and IFNγ ELISpot was performed. Phytohaemagglutinin (PHA, Sigma Aldrich), the HLA-A*24:02 restricted EBV epitope TYPVLEEMF (EBV BRLF1_198-206), or a pool of 18 viral peptides of different HLA restrictions were used as positive control. HLA-A*24:02 restricted PP1G peptide KYPENFFLL (HUMAN PP1G_113-121) or medium alone served as negative controls. Spot counts were determined using an ImmunoSpot S6 Analyzer (CTL, Shaker Heights, Ohio, USA) with T cell responses considered to be positive, if the mean number of spots per well was at least 10 and more than three times the mean number of spots of the negative controls.

### TCGA dataset analysis

The inventors downloaded the gene expression RNASeq data of LGGs and GBMs (Illumina) and the associated clinical data from the NIH National Cancer Institute GDC portal (https://portal.gdc.cancer.gov) released by TCGA. The survival data from TCGA were merged with gene expression data and other associated clinical information using corresponding sample ID.

### Statistical analyses

Statistical significance was calculated using two-way ANOVA followed by Tukey's multiple comparison test, one-way ANOVA with Dunnett's multiple comparison test or with multiple t-test, as suitable and respectively indicated. All values are expressed as mean ± standard error of the mean (SEM).

### 2. Results

Glioblastoma is an aggressive primary tumor of the central nervous system with a median overall survival in the range of 1.5 years despite multimodal treatment regimens. Novel therapeutic strategies are urgently needed. Oncolytic measles virus (MeV) provides an exciting opportunity in this regard. The inventors investigated here the combination of MeV with conventional therapies and identified that chemotherapy, virotherapy, and radiotherapy (CT-VT-RT) as a treatment sequence exhibits synergistic anti-glioma effects. Using RNA sequencing and immunopeptidome analysis with subsequent validations, the inventors characterized the underlying molecular mechanisms. CT-VT-RT initiated a type 1 interferon-response along with canonical Janus kinase/ signal transducers and activators of transcription (JAK/STAT) signaling and interferon-stimulated genes expression resulting in the activation of apoptotic cascades. Moreover, the inventors identified novel immunogenic peptides presented in the HLA ligandome of MeV-infected glioma cells. The inventors' data can be exploited for the generation of novel tailored immunovirotherapeutic strategies combining MeV and personalized peptide vaccinations.

Observations of complete remission in one patient treated with oncolytic measles virus in relapsing drug-refractory myeloma exemplifies proof of concept for efficacy. The MeV-initiated oncolytic cascade results in tumor cell lysis releasing virions and cellular debris encompassing highly immune-stimulatory pathogen-associated and danger-associated motif patterns (PAMPs and DAMPs) providing cues for the induction of antiviral and anti-tumoral immune responses.

Here, the inventors asked the questions (i) whether MeV can enhance therapeutic efficacy as part of a sequential combination treatment with radiation therapy (RT), temozolomide (TMZ), or lomustine chemotherapy; and if so, (ii) whether MeV-containing treatments induce molecular and immunological signatures that could be exploited to design tailored therapeutic strategies, e.g. to direct immune responses to MeV-infected gliomas. This is particularly relevant in the context of previous studies indicating the benefit of combining MeV with immune checkpoint inhibitors.

MeV induces autophagy and thereby sustains an anti-apoptotic environment by preventing cytochrome-C release to maintain its replication. The inventors cell lines expressed the MeV entry receptor CD46 (Fig. 1). The inventors then verified MeV-induced autophagy in LNZ308 and primary glioblastoma cells T1094/17 through immunoblotting and fluorescence microscopy by using a GFP-tagged LC3 (LC3-GFP) (Fig. 2). However, RT rather induces apoptosis, and in MeV-containing combination treatments starting with RT, the inventors observed a rather antagonistic "antiviral" effect as demonstrated by acute cytotoxicity assays with virotherapy (VT) and RT in three sequential regimens (Fig. 3). The VT-RT regimen showed significantly lower cell viability (Fig. 3) confirming previous results. Consequently, for the design of triple combinations of MeV, RT, and chemotherapy (CT), the inventors only considered combination treatments, where virotherapy precedes radiotherapy. This led to the following possible combination regimen: (i) virotherapy → radiotherapy → chemotherapy (VT-RT-CT) (ii) virotherapy → chemotherapy → radiotherapy (VT-CT-RT) and (iii) chemotherapy → virotherapy → radiotherapy (CT-VT-RT).

The inventors investigated these regimens thoroughly in LN229, LNZ308, GS8, and TMZ-resistant LN229 (R-LN229) cells utilizing TMZ or lomustin in combination with MeV and radiotherapy. The inventors identified CT-VT-RT as the most synergistic regimen (Figs. 4-11). The effect of virotherapy was most prominent in CT-VT-RT, with maximal cell death despite the delay of viral infection by 12 h in comparison to VT-initiated regimen, VT-RT-CT or VT-CT-RT (Figs. 5, 10) in LN229 or LNZ308 cells. Similar experimental conditions in glioma stem-like cells, GS8 revealed CT-VT-RT as the only triple regimen to elicit synergy (Fig. 7). Furthermore, the synergistic effect in CT-VT-RT was retained when substituting TMZ by lomustine (Fig. 10), or in the TMZ-resistant cell line R-LN229 (Fig. 11, red line).

The inventors next aimed at understanding the molecular and immunological cascade induced by CT-VT-RT. To this end, the inventors performed RNA sequencing and immunopeptidome analyses. The experimental design included monotherapies, dual, and synergistic triple regimen along with suitable controls at defined time points (Fig. 12). Upon processing of the raw sequencing data and ensuing statistical analysis, the inventors identified 2,592 genes with significant expression profile changes induced predominantly by regimen with MeV-GFP (Fig. 17) in the inventors' experimental setting.

All MeV-containing regimens (VT, CT-VT, CT-VT-RT) up-regulated RIG-I like receptor (RLR) signalling pathway genes upon detection of MeV RNA genomes (Fig. 17). The innate RNA sensor DEAD box protein-58 (DDX58) showed a 100-fold increase in mRNA levels at 72 hpt in VT, CT-VT, and CT-VT-RT regimen (Fig. 18a), resulting in transcription of interferon-beta (iFN-β) at 96 hpt with a 1000-fold increase in mRNA levels in these groups (Fig. 18a). Furthermore, our transcriptional data correlated with secreted amounts of IFN-β protein with detection of 123 pg/ml, 113 pg/ml and 103 pg/ml of IFN-β per microgram of whole protein in VT, CT-VT and CT-VT-RT regimen, respectively (Fig. 18b), but no detectable IFN-β in other treatment regimen.

The binding of secreted IFN-β to the interferon α-β receptor (IFNAR) triggers the downstream canonical JAK-STAT signaling. The inventors observed a 6-fold increase in STAT1 mRNA in VT and CT-VT regimen at 72 hpt (Fig. 18a), but a similar increase only at 96 hpt in CT-VT-RT (Fig. 18a). These data indicate a delayed STAT1 signaling for the latter, allowing for efficient viral proliferation in the absence of host antiviral responses. The STAT signaling resulting in transcription of interferon stimulated genes (ISGs) indicate a distinct antiviral state with a 100-fold increase in interferon-induced 15 kDa protein (ISG15), and interferon-induced protein with tetratricopeptide-repeats 2 (IFIT2) mRNA levels (Fig. 18a), while a 1,000-fold increase was observed in myxoma resistance protein 1 (MX1), and 2 (MX2), 2'-5'-oligoadenylate synthase 1 (OAS1) and 2 (OAS2) mRNAs in all virus-containing regimen (Fig. 18a). The antiviral ISGs possess the ability to initiate apoptosis apart from their innate antiviral responses. The inventors observed activation of TNF-related apoptosis-inducing ligand (TRAIL) triggering apoptotic cascade only in virus-containing regimen (Fig. 17). The immunoblots showed enhanced TRAIL in CT-VT or CT-VT-RT compared to VT, alone, at 72 hpt and 96 hpt (Fig. 18c, middle panel). TRAIL expression corresponded to Caspase 3/7 activity with a 3-fold increase observed in CT-VT at 72 hpt and a more than 2-fold increase during the CT-VT-RT regimen at 72 hpt and 96 hpt in comparison to a maximal 1.5-fold increase in VT, alone (Fig. 18d).

The inventors identified several interferon-related genes, which were distinctively upregulated upon MeV infection (Fig. 17). The sterile alpha motif domain-containing protein 9 (SAMD9) was expressed at basal levels in LNZ308 (0 h) with no change in expression upon TMZ, DMSO, or radiation treatments (Fig.18c, upper panel). However, there was a significant increase in SAMD9 protein expression upon MeV infection at 72 hpt and 96 hpt in all virus-containing regimen, with CT-VT-RT showing higher expression in comparison to VT, alone (Fig. 18c, upper panel). Similarly, the inventors observed overexpression of anti-tumoral host factor basic leucine zipper transcriptional factor ATF2-like protein (BATF2) in all virus-containing regimen (Fig. 17). Yet, the increase in mRNA expression did not correlate with BATF2 protein levels with basal level (0 h) of BATF2 expression detected in all regimen (Fig.18e, upper panel) during the course of measurement until 96 hpt.

Upregulated C-X-C motif chemokine-10 (CXCL10) transcription in CT-VT-RT (Fig. 17) correlated with significantly increased protein expression (Fig. 18b) with 633.9 pg/ml of CXCL10 per microgram of total protein detected by ELISA, in comparison to just 289.6 pg/ml using only CT-VT without RT. Interestingly, the leukocyte migratory C-C motif chemokine-57 (CCL5) was uniquely expressed in MeV-containing regimen (Figs. 17, 18b). VT elicited a maximal secretion of CCL5 with 45.2 pg/ml of this cytokine per microgram of total protein, while CT-VT and CT-VT-RT triggered lower expression of CCL5 with 25.7 pg/ml and 32.5 pg/ml, respectively (Fig. 18b). The potent immune-stimulatory and chemoattractant abilities of both of these chemokines might trigger a MeV-induced, host-directed immune response with eventual anti-glioma activity. Based on the RNASeq data (Fig. 17) and the validation experiments (Fig. 18), the inventors schematically summarized the molecular cascade (Fig. 19) and consequently continued with inhibitors of IFN-β production (BX795) and STAT1 signalling (fludarabine) (Fig. 19, red circles) in the CT-VT-RT regimen.

STAT1 transcription and protein phosphorylation was downregulated by fludarabine and by BX795 alone or in combination with latter applied after CT-VT-RT treatment (Figs. 20a,b). IFN-β secretion was abolished in CT-VT-RT plus BX795, in CT-VT-RT plus BX795 plus fludarabine, and also in CT-VT-RT plus fludarabine (Figs. 20a,c). These data suggest interdependency with STAT1 in the setting. Inhibition of the IFN-β-STAT1 axis resulted in a complete loss of ISGs at the transcriptional level, along with an absence of TRAIL expression (Figs. 20a,d, lower panel). Yet, SAMD9 expression was not impaired upon inhibition of the IFN-β - STAT1 pathway (Fig. 20d, upper panel). Most interestingly, BATF2 was highly upregulated in IFN-β and STAT1-inhibited samples in comparison to CT-VT-RT alone or CT-VT-RT + DMSO, with a corresponding increase in interferon regulatory factor 1 (IRF1) expression (Fig. 20b). The inventors therefore used proximity ligation assays and identified a possible molecular interaction of BATF2 with IRF1 with an increase in red amplification signals (Fig. 20e) distinctly, in treatment with CT-VT-RT plus fludarabine. The inventors also observed a decrease of CCL5 in CT-VT-RT plus fludarabine in comparison to CT-VT-RT, alone (Fig. 20f), while IFN-β was completely absent. This is particularly relevant, because CCL5 is a known BATF2 - IRF1 induced gene. The observed correlation in CCL5 expression in accordance with BATF2 and IRF1 expression in the setting therefore suggests a BATF2-IRF1 interaction-dependent transcription of CCL5. Moreover, the inventors observed caspase activity similar to CT-VT-RT despite the abrogation of TRAIL expression in CT-VT-RT plus BX795, (Fig. 20g) demonstrating the induction of an alternative apoptotic machinery by the substitutive BATF2-IRF1 interaction.

Of note, CT-VT-RT increased the cellular capacity of antigen presentation with 1.5- to 2-fold elevated expression of MHC I, A (HLA-A) and B (HLA-B) mRNA at 96 hpt in comparison to CT-VT or VT (Fig. 18a). In addition, the transcript of antigen processing protein TAP1 was also upregulated in all virus-containing regimens (Fig. 18a). The inventors therefore reasoned that CT-VT-RT might stimulate the presentation of tumor antigens by glioma cells. In fact, the immunopeptidome analysis revealed that CT-VT and CT-VT-RT distinctively increased antigen presentation with 1,430 and 1,222 peptides isolated from MHC-molecules after treatment, respectively (Figs. 13, 14). Among these, we isolated a tumor-associated peptide (LYTDRTEKL; SEQ ID NO: 11) processed from transforming growth factor-beta-induced (TGFBI), exclusively presented in CT-VT. Based on the TCGA dataset interrogation, TGFBI was significantly overexpressed in glioblastoma compared to lower grade gliomas (Fig. 15). Furthermore, other TGFBI-derived peptides from glioblastoma samples have been previously discovered and shown to be immunogenic. Thus, the LYTDRTEKL (SEQ ID NO:11) peptide could be exploited to further enhance the efficacy of CT-VT-RT regimen by a peptide vaccination using this sequence to augment a directed immune response.

The inventors further identified the presentation of a novel HLA-A*24-restricted MeV peptide (Fig. 16), VYPRYSNFI (SEQ ID NO: 1), processed from a highly conserved viral region, the MeV-L polymerase, in all virus-containing regimen. This peptide was particularly increased in the triple regimen CT-VT-RT (2.7-fold increase), compared with MeV alone (Fig. 20h). The MeV-L peptide resulted in significant IFN-γ secretion with a mean spot count of 264.5 measured using a 12-day recall IFN-γ ELISPOT assay (Fig. 20i), demonstrating that the identified viral peptide is highly immunogenic. The induction of this VYPRYSNFI (SEQ ID NO:1) peptide by the inventors' triple regimen might be clinically relevant in two ways: First, MeV-infected cells create a specific actionable tumor-associated immunogenic signature. Second, the peptide could be exploited for a tailored vaccination strategy that enhances immune responses towards remaining MeV-infected cells that are resistant to oncolysis. These observed immunogenic signatures in CT-VT-RT-treated cells were accompanied with an increase in PD-1L transcription (Fig. 17) supporting previously reported benefit from combining MeV with immune checkpoint blockade inhibitors.

In conclusion, a triple sequential combination of TMZ (or lomustine), MeV, and radiation therapy has synergistic anti-glioma activity and induces an effectuating molecular and immunological signature. This regimen could be further enhanced by tailored peptide vaccinations with these newly identified and treatment-induced peptide sequences, potentially in combination with checkpoint blockade antibodies. Of note, regulatory preparations might be facilitated due to the existing practice of utilizing Measles virus (MeV) as vaccines. Taken together, the inventors' data suggest the design of an innovative personalized therapeutic strategy for a subset of patients, in particular glioblastoma patients.

### 3. Supplementary results

### MeV receptor CD46 is expressed on glioma cells, glioma stem-like cells and primary 342 glioblastoma cell lines

Membrane cofactor protein or CD46 serves as one receptor for the entry of vaccine strain measles viruses. CD46 is expressed on all human nucleated cells, but frequently upregulated in tumors including glioblastoma. The inventors characterized the different glioma cell lines for CD46 expression via flow cytometry with mean fluorescence intensity coefficient (MFI, CD46/lgG) > 10 in all cell lines except primary GBM T708/16, and the highest expression observed in glioma cell lines LNZ308 and LN229 (Fig. 1a). Glioma stem-like cells GS3 and GS8 and primary glioblastoma cells T81/16, T708/16, and T1094/17 expressed CD46 at comparably lower but clearly detectable levels. Yet, all cell lines revealed susceptibility to MeV as observed using virus-encoded GFP-expression via fluorescence microscopy (Fig. 1b). All lines were negative for Nectin-4 expression (data not shown), an alternative MeV entry receptor expressed on the respiratory epithelium, but also in breast and ovarian cancer.

### Modulation of CD46 expression in LNZ308 by hypoxia and temozolomide, but not by irradiation

The inventors next assessed CD46 expression in LN229 and LNZ308 cells after culture at 1% O₂ (hypoxia) or treatments with irradiation or temozolomide (TMZ) (Fig. 1c-e). The expression of CD46 increased in LNZ308 cells after culturing in hypoxia (Fig. 1c), but remained unchanged after radiotherapy in both cell lines (Fig. 1d). Low doses of TMZ, i.e. 10 µM and 100 µM, increased CD46 expression in LNZ308 cells, while treatment with 1,000 µM of TMZ significantly lowered CD46 levels (Fig. 1e).

### Combined treatments of virotherapy and radiation therapy are only synergistic when radiation therapy is administered after virotherapy

Due to its relevance for the current clinical scenario, the inventors combined virotherapy (VT) with irradiation (RT) and chemotherapy (CT) to identify a chronological synergistic regimen elicited by such a treatment strategy. The induction of autophagy including mitophagy upon MeV infection prevents release of cytochrome C, thereby inhibiting apoptosis. This suggests that initiation of treatment with irradiation, an established apoptosis inducer, will likely lead to an antagonistic "antiviral" effect when followed by virotherapy. The inventors confirmed this hypothesis through cytotoxicity assays with MeV and γ-irradiation performed in three sequential regimens: Irradiation followed by virus (RT-VT), virus followed by irradiation (VT-RT), and simultaneous treatment with both (Sim Trt). The treatment regimen initiated with virotherapy followed by irradiation (VT-RT) showed significantly lower cell viability than both other regimen (Fig. 3) in accordance with previous results. RT-VT resulted in higher cell survival compared to other regimen indicating an unfavourable role of irradiation to viral proliferation, when applied first. As a consequence, the inventors incorporated alkylating chemotherapy, i.e. TMZ, to these treatment schedules such that virotherapy always precedes radiotherapy, resulting in three possible combination regimen: (i) virotherapy → radiotherapy → temozolomide (ii) virotherapy → temozolomide → radiotherapy, and (iii) temozolomide → virotherapy → radiotherapy.

### Triple therapies including virotherapy, radiotherapy and temozolomide are synergistic if the treatment algorithm starts with temozolomide and is followed by virotherapy and radiotherapy

The inventors carried out cytotoxic survival assays in glioma cell lines, LN229 and LNZ308, utilising TMZ in combination with virotherapy and radiotherapy as monotherapies, dual or triple regimen. The inventors calculated predictive values employing the Chou-Talalay fractional product method. LN229 and LNZ308 cells were treated with TMZ based on their EC50 values described previously and administered doses of 0 µM, 10% EC50, 50% EC50 and EC50. The monotherapy with γ-irradiation and TMZ clearly revealed a dose-dependent response in both cell lines (Fig. 4). LN229 had lower cell viability with increasing doses of radiation and temozolomide which could be due to the mutated but active p53 expression in comparison to the p53 null LNZ308. Conversely, lower cell viability was observed in LNZ308 upon virotherapy (Fig. 4). All triple regimen elicited dose-dependency with synergistic effects (indicated by red arrows) mainly observed in CT-VT-RT in both cell lines (Fig. 5).

The effect of virotherapy was most prominent in CT-VT-RT (Fig. 5a,b) with maximal cell death over all regimens despite the delay of viral infection by 12 h in comparison to VT-initiated regimens, VT-RT-CT and VT-CT-RT (Fig. 5c-f). The treatment with double therapy, CT-VT (CT-VT-RT with RT = 0 Gy) can elicit synergy at low doses of 10% EC50 of TMZ in combination with 0.01 MOI and 0.05 MOI of MeV in both cell lines, as does VT-CT (Fig. 5c,d). However, the synergy associated when combining TMZ with MeV is lost in VT-CT when applying 50% EC50 of TMZ in LN229 (Fig. 5). In contrast, the synergistic effect was sustained in CT-VT and a similar effect more evident with the highest dose of TMZ (EC50) in both cell lines (Fig. 5). The additional cytotoxicity due to γ-irradiation was evident in VT-RT-CT treatment sequence (Fig. 5e,f), while found limited in both CT-VT-RT and VT-CT-RT, possibly due to delayed radiation treatment (12 h later). The dose-dependent effect of TMZ in the highest virus dose (0.05 MOI) and radiation dose (4 Gy) is lost in both cell lines in non-synergistic regimens, VT-CT-RT and VT-RT-CT (Fig. 5c-f). In contrast, dose-dependent decline in cell viability was sustained in CT-VT-RT, although an additive rather than a synergistic effect became evident, here (Fig. 5a,b).

The ultimate goal for synergistic treatments with combination of agents is to elicit maximal therapeutic effect with minimal toxic doses in patients. Given the clinical routine for treatment of glioblastoma with fractionated individual doses up to 2 Gy irradiation, the inventors identified CT-VT-RT as the only regimen to exhibit a synergistic effect at 2 Gy in both LN229 and LNZ308 at 10% EC50 and 50% EC50 of TMZ along with 0.05 MOI of MeV-GFP (Fig. 5). The pronounced oncolytic effect observed in the most synergistic regimen initiated with chemotherapy over the VT-initiated triple regimen suggests that initial TMZ treatment established an environment conducive for viral proliferation. The observed increase in CD46 receptor expression subjected to low doses of TMZ (Fig. 1e) through flow cytometric analysis might be one factor in augmenting viral infectivity.

### CT-VT-RT effected synergy in glioma stem-like cell, GS8

The inventors determined the EC50 of TMZ in GS8 to be 250 µM (Fig. 6) using acute cytotoxicity assays as described. Employing selective doses identified synergistic in LN229 and LNZ308 the inventors performed cytotoxic survival assays with 2 Gy irradiation, 0.05 MOI of MeV and 10% EC50 of TMZ in previously established combinations (Fig. 7).

### CT-VT-RT induced synergy was sustained upon substitution of temozolomide with lomustine

The inventors determined the EC₅₀ of lomustine in LN229 and LNZ308 cells to be 16 µM and 45 µM respectively, using acute cytotoxicity assays (Fig. 8). The monotherapies with γ-irradiation and MeV effected dose responses as expected (Fig. 9) and in accordance with cell viability observed previously (Fig. 4). The CT-VT-RT initiated synergistic effect was retained when substituting TMZ by lomustine/CCNU. CT-VT-RT using CCNU as chemotherapeutic agent revealed a significant synergistic effect even with maximal doses of irradiation (4 Gy) (Fig. 10), in contrast to TMZ (Fig. 5). The cell viability and dose dependence in the non-synergistic regimens were similar to those previously observed in triple regimens using TMZ (Fig. 5).

Furthermore, the inventors treated TMZ-resistant LN229 (R-LN229) cells with CT-VT-RT employing lomustine, the second line alkylating agent used in patients with progressive glioblastoma, in combination with clinically relevant radiation doses of 1 Gy and 2 Gy along with MeV-GFP. The R-LN229 cells displayed higher cell viability than LN229 in the absence of CCNU (0 µm or DMSO) despite receiving treatment with irradiation and MeV (Fig. 11, blue lines). However, both R-LN229 and parental LN229 cells exhibited comparable levels of susceptibility to MeV infection. Strikingly, treatment with synergistic CT-VT-RT resulted in killing of R-LN229 cells similar to parental LN229 cells (Fig. 11, red lines) thereby rescuing the proliferative effect. The initiation of treatment with alkylating chemotherapy followed sequentially with MeV and γ-irradiation (CT-VT-RT) was synergistic in glioma cells and in chemo-resistant cells with pronounced induction of viral proliferative mechanisms resulting in an enhanced oncolytic cascade effect.

### Selective inhibition of TBK1/IKKε or STAT1 -signalling abrogates TRAIL expression

The inventors aimed to further characterize the role of the RLR - IFN-β-STAT1 signalling axis by using the compound BX795 to selectively inhibit Tank binding kinase-1 (TBK1)/IkappaB kinase epsilon (IKKε) signalling that should downregulate IFN-β (Fig. 19a, red circle). Moreover, the inventors used fludarabine to inhibit downstream STAT1-signalling (Fig. 19b, red circle). The transcription of STAT1 was indeed downregulated (Fig. 20a) by both substances as well as by the combination post CT-VT-RT treatment, most likely due to inhibition of the positive feedback loop via IFNAR. The inventors have previously shown the absence of RLR and downstream type-1 interferon signalling in CTL (R/V) regimen (Figs. 18a and 18b) and the inventors hence, not pursued in this experimental setting with molecular inhibitors. The inhibition of STAT1-signalling was further validated through immunoblot analysis with complete loss of phospho-STAT1 (Y701) in treatments with CT-VT-RT plus fludarabine, CT-VT-RT plus BX795, and CT-VT-RT plus BX795 plus fludarabine. In contrast, phospho-STAT1 was visible in CT-VT-RT, alone, and CT-VT-RT plus DMSO (Fig. 20b). However, despite the absence of phospho-STAT1 we identified similar levels of total STAT1 protein in all treatments besides cells treated with CT-VT-RT plus fludarabine that revealed increased amounts of STAT1 protein (Fig. 20b). These increased levels of total STAT1 when subjected to fludarabine, alone, could be due to intact upstream signaling with induction of IFN-β and subsequent STAT1 transcription, but lack of phosphorylation thereof. Thereby, activation of downstream transcriptional and autocrine signaling events via the IFNAR feedback loop would be prevented. Furthermore, CT-VT-RT activated viral RNA-dependent RLR signaling through upregulation of DDX58 mRNA levels (Fig. 20a) with production of IFN-β corresponding to mRNA levels with 158 pg/ml and 226 pg/ml of IFN-β protein (Fig. 20c) detected in CT-VT-RT and CT-VT-RT plus DMSO, respectively. The secretion of IFN-β correlates with STAT1 phosphorylation and the stimulation of transcription of downstream ISGs as evidenced by increased levels of MX1 and ISG15 mRNA (Fig. 20a). However, there was a complete loss of IFN-β production not only in treatments with CT-VT-RT plus BX795 and CT-VT-RT plus BX795 plus fludarabine, but also in CT-VT-RT plus fludarabine (Figs. 20a and 20c). These data point at the autocrine role of STAT1 in upstream inhibition of IFN-β in this setting. Moreover, the inhibition of IFN-β - STAT1 - axis results in a complete loss of ISGs at the transcriptional level (Fig. 20a), along with the absence of TRAIL expression (Fig. 20d, lower panel) in these regimen. Thereby, the pro-inflammatory and anti-tumoral activity of IFN-β is further demonstrated. However, the absence of TRAIL expression did not result in loss of caspase activity, but rather decreased activity (Fig. 20g) comparable to levels previously noticed in virotherapy alone (Fig. 18d), thereby further demonstrating the existence of at least one alternative apoptotic mechanism of tumor cell killing.

## Claims

1. A pharmaceutical composition for the treatment and/or prophylaxis of a disease comprising a first peptide derived from the RNA-directed RNA polymerase L of the Measles virus (MeV-L polymerase).

2. The pharmaceutical composition of claim 1, **characterized in that** said first peptide is derived from the MeV-L polymerase of the Edmondston strain of MeV.

3. The pharmaceutical composition of claim 1 or 2, wherein said first peptide comprises at least nine sequentially concatenated amino acids derived from the MeV-L polymerase.

4. The pharmaceutical composition of any of the preceding claims, wherein said first peptide comprises the following amino acid sequence:
*X₁* Y-P-R-Y-S-N-F-*X₂*,
wherein
*X₁* and *X₂* are selected from the group consisting of: V, I, and L.

5. The pharmaceutical composition of any of the preceding claims, **characterized in that** said first peptide comprises the following amino acid sequence V-Y-P-R-Y-S-N-F-I (SEQ ID NO: 1).

6. The pharmaceutical composition of any of the preceding claims comprising a second peptide comprising the following amino acid sequence:
*X₁*-Y-T*-X₂*-R-T-*X₃-*K*-X₄*,
wherein
- *X₁* is selected from the group consisting of: L, I, and V;
- X₂ is D or E;
- *X₃* is E or D;
- *X₄* is selected from the group consisting of: L, I, and V.

7. The pharmaceutical composition of claim 5, **characterized in that** said second peptide comprises the following amino acid sequence L-Y-T-D-R-T-E-K-L (SEQ ID NO: 11).

8. The pharmaceutical composition of any of the preceding claims, **characterized in that** said disease is a tumor disease, preferably a glioblastoma, wherein preferably said pharmaceutical composition is a tumor vaccine, further preferably a vaccine against glioblastoma.

9. The pharmaceutical composition of any of the preceding claims, **characterized in that** it is configured for an administration to a patient in need, preferably to a patient with an HLA type HLA-A*24, further preferably with an HLA type HLA-A*24:03, further preferably it is configured for an administration after said patient has been treated with an oncolytic Measles vaccination virus, and further preferably it is configured for an administration followed by the additional administration of an immune checkpoint inhibitor.

10. An isolated peptide for use in the therapy and/or prophylaxis of a disease, preferably a tumor disease, **characterized in that** said peptide is the first peptide of the pharmaceutical composition of any of claims 1-9.

11. An isolated peptide for use in the therapy and/or prophylaxis of a disease, preferably a tumor disease, **characterized in that** said peptide is the second peptide of the pharmaceutical composition of any of claims 6-9.

12. A nucleic acid encoding a peptide of claim 10 or 11, optionally linked to a promoter sequence.

13. An expression vector capable of expressing the nucleic acid of claim 12.

14. A recombinant host cell comprising the peptide of claim 10 or 11, the nucleic acid of claim 12, or the expression vector of claim 13, wherein said host cell is preferably an antigen presenting cell, further preferably a dendritic cell.

15. An antibody and/or T cell receptor and/or chimeric antigen receptor T cells which specifically recognize the peptide of claim 10 or 11.


SEQUENCE LISTING

<110> Eberhard Karls Universität Medizinische Fakultät

<120> Immunotherapeutic peptides

<130> 5402P578EP

<160> 72

<170> BiSSAP 1.3.6

<210> 1
<211> 9
<212> PRT
<213> Artificial Sequence

<220>
<223> Immunotherapeutic peptide

<400> 1
Val Tyr Pro Arg Tyr Ser Asn Phe Ile
1               5

<210> 2
<211> 9
<212> PRT
<213> Artificial Sequence

<220>
<223> Immunotherapeutic peptide

<400> 2
Val Tyr Pro Arg Tyr Ser Asn Phe Leu
1               5

<210> 3
<211> 9
<212> PRT
<213> Artificial Sequence

<220>
<223> Immunotherapeutic peptide

<400> 3
Ile Tyr Pro Arg Tyr Ser Asn Phe Ile
1               5

<210> 4
<211> 9
<212> PRT
<213> Artificial Sequence

<220>
<223> Immunotherapeutic peptide

<400> 4
Leu Tyr Pro Arg Tyr Ser Asn Phe Ile
1               5

<210> 5
<211> 9
<212> PRT
<213> Artificial Sequence

<220>
<223> Immunotherapeutic peptide

<400> 5
Leu Tyr Pro Arg Tyr Ser Asn Phe Leu
1               5

<210> 6
<211> 9
<212> PRT
<213> Artificial Sequence

<220>
<223> Immunotherapeutic peptide

<400> 6
Ile Tyr Pro Arg Tyr Ser Asn Phe Leu
1               5

<210> 7
<211> 9
<212> PRT
<213> Artificial Sequence

<220>
<223> Immunotherapeutic peptide

<400> 7
Val Tyr Pro Arg Tyr Ser Asn Phe Val
1               5

<210> 8
<211> 9
<212> PRT
<213> Artificial Sequence

<220>
<223> Immunotherapeutic peptide

<400> 8
Ile Tyr Pro Arg Tyr Ser Asn Phe Val
1               5

<210> 9
<211> 9
<212> PRT
<213> Artificial Sequence

<220>
<223> Immunotherapeutic peptide

<400> 9
Ile Tyr Pro Arg Tyr Ser Asn Phe Val
1               5

<210> 10
<211> 9
<212> PRT
<213> Artificial Sequence



<220>
<223> Immunotherapeutic peptide

<400> 10
Leu Tyr Pro Arg Tyr Ser Asn Phe Val
1               5

<210> 11
<211> 9
<212> PRT
<213> Artificial Sequence



<220>
<223> Immunotherapeutic peptide

<400> 11
Leu Tyr Thr Asp Arg Thr Glu Lys Leu
1               5

<210> 12
<211> 9
<212> PRT
<213> Artificial Sequence



<220>
<223> Immunotherapeutic peptide

<400> 12
Ile Tyr Thr Asp Arg Thr Glu Lys Leu
1               5

<210> 13
<211> 9
<212> PRT
<213> Artificial Sequence



<220>
<223> Immunotherapeutic peptide

<400> 13
Leu Tyr Thr Asp Arg Thr Glu Lys Ile
1               5

<210> 14
<211> 9
<212> PRT
<213> Artificial Sequence

<220>
<223> Immunotherapeutic peptide

<400> 14
Ile Tyr Thr Asp Arg Thr Glu Lys Ile
1               5

<210> 15
<211> 9
<212> PRT
<213> Artificial Sequence

<220>
<223> Immunotherapeutic peptide

<400> 15
Leu Tyr Thr Glu Arg Thr Glu Lys Leu
1               5

<210> 16
<211> 9
<212> PRT
<213> Artificial Sequence

<220>
<223> Immunotherapeutic peptide

<400> 16
Leu Tyr Thr Asp Arg Thr Asp Lys Leu
1               5

<210> 17
<211> 9
<212> PRT
<213> Artificial Sequence

<220>
<223> Immunotherapeutic peptide

<400> 17
Leu Tyr Thr Glu Arg Thr Asp Lys Leu
1               5

<210> 18
<211> 9
<212> PRT
<213> Artificial Sequence

<220>

<223> Immunotherapeutic peptide

<400> 18
Ile Tyr Thr Glu Arg Thr Glu Lys Leu
1               5

<210> 19
<211> 9
<212> PRT
<213> Artificial Sequence


<220>
<223> Immunotherapeutic peptide

<400> 19
Ile Tyr Thr Asp Arg Thr Asp Lys Leu
1               5

<210> 20
<211> 9
<212> PRT
<213> Artificial Sequence


<220>
<223> Immunotherapeutic peptide

<400> 20
Ile Tyr Thr Glu Arg Thr Asp Lys Leu
1               5

<210> 21
<211> 9
<212> PRT
<213> Artificial Sequence


<220>
<223> Immunotherapeutic peptide

<400> 21
Leu Tyr Thr Glu Arg Thr Glu Lys Ile
1               5

<210> 22
<211> 9
<212> PRT
<213> Artificial Sequence


<220>
<223> Immunotherapeutic peptide

<400> 22
Leu Tyr Thr Asp Arg Thr Asp Lys Ile
1               5

<210> 23
<211> 9

<212> PRT
<213> Artificial Sequence

<220>
<223> Immunotherapeutic peptide

<400> 23
Leu Tyr Thr Glu Arg Thr Asp Lys Ile
1               5

<210> 24
<211> 9
<212> PRT
<213> Artificial Sequence

<220>
<223> Immunotherapeutic peptide

<400> 24
Ile Tyr Thr Glu Arg Thr Glu Lys Ile
1               5

<210> 25
<211> 9
<212> PRT
<213> Artificial Sequence

<220>
<223> Immunotherapeutic peptide

<400> 25
Ile Tyr Thr Asp Arg Thr Asp Lys Ile
1               5

<210> 26
<211> 9
<212> PRT
<213> Artificial Sequence

<220>
<223> Immunotherapeutic peptide

<400> 26
Ile Tyr Thr Glu Arg Thr Asp Lys Ile
1               5

<210> 27
<211> 9
<212> PRT
<213> Artificial Sequence

<220>
<223> Immunotherapeutic peptide

<400> 27

Val Tyr Thr Asp Arg Thr Glu Lys Leu
1               5

<210> 28
<211> 9
<212> PRT
<213> Artificial Sequence

<220>
<223> Immunotherapeutic peptide

<400> 28
Ile Tyr Thr Asp Arg Thr Glu Lys Val
1               5

<210> 29
<211> 9
<212> PRT
<213> Artificial Sequence

<220>
<223> Immunotherapeutic peptide

<400> 29
Leu Tyr Thr Asp Arg Thr Glu Lys Val
1               5

<210> 30
<211> 9
<212> PRT
<213> Artificial Sequence

<220>
<223> Immunotherapeutic peptide

<400> 30
Val Tyr Thr Asp Arg Thr Glu Lys Val
1               5

<210> 31
<211> 9
<212> PRT
<213> Artificial Sequence

<220>
<223> Immunotherapeutic peptide

<400> 31
Ile Tyr Thr Glu Arg Thr Glu Lys Val
1               5

<210> 32
<211> 9
<212> PRT
<213> Artificial Sequence

<220>
<223> Immunotherapeutic peptide

<400> 32
Leu Tyr Thr Glu Arg Thr Glu Lys Val
1               5

<210> 33
<211> 9
<212> PRT
<213> Artificial Sequence

<220>
<223> Immunotherapeutic peptide

<400> 33
Val Tyr Thr Glu Arg Thr Glu Lys Val
1               5

<210> 34
<211> 9
<212> PRT
<213> Artificial Sequence

<220>
<223> Immunotherapeutic peptide

<400> 34
Val Tyr Thr Glu Arg Thr Glu Lys Ile
1               5

<210> 35
<211> 9
<212> PRT
<213> Artificial Sequence

<220>
<223> Immunotherapeutic peptide

<400> 35
Val Tyr Thr Asp Arg Thr Asp Lys Val
1               5

<210> 36
<211> 9
<212> PRT
<213> Artificial Sequence

<220>
<223> Immunotherapeutic peptide

<400> 36
Ile Tyr Thr Asp Arg Thr Asp Lys Val
1               5

<210> 37
<211> 9
<212> PRT
<213> Artificial Sequence

<220>
<223> Immunotherapeutic peptide

<400> 37
Leu Tyr Thr Asp Arg Thr Asp Lys Val
1               5

<210> 38
<211> 9
<212> PRT
<213> Artificial Sequence

<220>
<223> Immunotherapeutic peptide

<400> 38
Val Tyr Thr Asp Arg Thr Asp Lys Leu
1               5

<210> 39
<211> 9
<212> PRT
<213> Artificial Sequence

<220>
<223> Immunotherapeutic peptide

<400> 39
Val Tyr Thr Asp Arg Thr Asp Lys Ile
1               5

<210> 40
<211> 9
<212> PRT
<213> Artificial Sequence

<220>
<223> Immunotherapeutic peptide

<400> 40
Val Tyr Thr Glu Arg Thr Asp Lys Val
1               5

<210> 41
<211> 9
<212> PRT
<213> Artificial Sequence

<220>
<223> Immunotherapeutic peptide

<400> 41
Ile Tyr Thr Glu Arg Thr Asp Lys Val
1               5

<210> 42
<211> 9
<212> PRT
<213> Artificial Sequence

<220>
<223> Immunotherapeutic peptide

<400> 42
Leu Tyr Thr Glu Arg Thr Asp Lys Val
1               5

<210> 43
<211> 9
<212> PRT
<213> Artificial Sequence

<220>
<223> Immunotherapeutic peptide

<400> 43
Val Tyr Thr Glu Arg Thr Asp Lys Ile
1               5

<210> 44
<211> 9
<212> PRT
<213> Artificial Sequence

<220>
<223> Immunotherapeutic peptide

<400> 44
Val Tyr Thr Glu Arg Thr Asp Lys Leu
1               5

<210> 45
<211> 20
<212> DNA
<213> Artificial Sequence

<220>
<223> PCR primer

<400> 45
gaccacgatc ctctacaagc                                                   20

<210> 46
<211> 21
<212> DNA

<213> Artificial Sequence

<220>
<223> PCR primer

<400> 46
tcccacacag tgaagctgat g                                                21

<210> 47
<211> 23
<212> DNA
<213> Artificial Sequence

<220>
<223> PCR primer

<400> 47
agacaaagat gaagagagca gga                                              23

<210> 48
<211> 21
<212> DNA
<213> Artificial Sequence

<220>
<223> PCR primer

<400> 48
gctcggacat tgctgaagaa g                                                21

<210> 49
<211> 21
<212> DNA
<213> Artificial Sequence

<220>
<223> PCR primer

<400> 49
gagtattggg accaggagac a                                                21

<210> 50
<211> 22
<212> DNA
<213> Artificial Sequence

<220>
<223> PCR primer

<400> 50
cgtcgcagcc atacattatc tg                                               22

<210> 51
<211> 19
<212> DNA
<213> Artificial Sequence

<220>
<223> PCR primer

<400> 51
tgagatggga gccgtcttc                                                    19

<210> 52
<211> 20
<212> DNA
<213> Artificial Sequence

<220>
<223> PCR primer

<400> 52
ctacacatca cagcagcgac                                                   20

<210> 53
<211> 28
<212> DNA
<213> Artificial Sequence

<220>
<223> PCR primer

<400> 53
cggatataaa gaatgtaaca ttgttatc                                          28

<210> 54
<211> 23
<212> DNA
<213> Artificial Sequence

<220>
<223> PCR primer

<400> 54
atgagcatac tcctctggtt tca                                               23

<210> 55
<211> 20
<212> DNA
<213> Artificial Sequence

<220>
<223> PCR primer

<400> 55
cctccttggg ttcgtctaca                                                   20


<210> 56
<211> 20
<212> DNA
<213> Artificial Sequence


<220>
<223> PCR primer

<400> 56
ggctgatatc tgggtgccta                                                   20


<210> 57
<211> 21
<212> DNA
<213> Artificial Sequence


<220>
<223> PCR primer

<400> 57
gtctcctcca aattgctctc c                                                 21


<210> 58
<211> 22
<212> DNA
<213> Artificial Sequence


<220>
<223> PCR primer

<400> 58
cagtattcaa gcctcccatt ca                                                22


<210> 59
<211> 18
<212> DNA
<213> Artificial Sequence


<220>
<223> PCR primer

<400> 59
atgggctggg acctgacg                                                     18


<210> 60

<211> 19
<212> DNA
<213> Artificial Sequence

<220>
<223> PCR primer

<400> 60
gccgatcttc tgggtgatc                                                    19

<210> 61
<211> 21
<212> DNA
<213> Artificial Sequence

<220>
<223> PCR primer

<400> 61
cgctggtgct gaaactgaag a                                                 21

<210> 62
<211> 21
<212> DNA
<213> Artificial Sequence

<220>
<223> PCR primer

<400> 62
gcgatggcat tctgggcttt a                                                 21

<210> 63
<211> 21
<212> DNA
<213> Artificial Sequence

<220>
<223> PCR primer

<400> 63
agttcagaat ggagcagatg g                                                 21

<210> 64
<211> 22
<212> DNA
<213> Artificial Sequence

<220>
<223> PCR primer

<400> 64
accgaagact cattactggg aa                                                22

<210> 65
<211> 22
<212> DNA
<213> Artificial Sequence

<220>
<223> PCR primer

<400> 65
cacagaacta cagagagact tc                                                22

<210> 66
<211> 20
<212> DNA
<213> Artificial Sequence

<220>
<223> PCR primer

<400> 66
caagcataga ccgtcaggag                                                   20

<210> 67
<211> 23
<212> DNA
<213> Artificial Sequence

<220>
<223> PCR primer

<400> 67
gacttctccc aacctggata atg                                               23

<210> 68
<211> 22
<212> DNA
<213> Artificial Sequence

<220>
<223> PCR primer

<400> 68
ctgtcaatct gctctaggaa gc                                                22

<210> 69
<211> 22
<212> DNA
<213> Artificial Sequence

<220>
<223> PCR primer

<400> 69
cagaacagag aacacgagac ca                                                22


<210> 70
<211> 24
<212> DNA
<213> Artificial Sequence


<220>
<223> PCR primer

<400> 70
gttcagtgac attcagcaac tcta                                              24


<210> 71
<211> 22
<212> DNA
<213> Artificial Sequence


<220>
<223> PCR primer

<400> 71
aaagacactc aaccagaagg ag                                                22


<210> 72
<211> 21
<212> DNA
<213> Artificial Sequence


<220>
<223> PCR primer

<400> 72
gcccaccaat gtagaggatt c                                                 21